# EUROPEAN PATENT APPLICATION

(11) **EP 2 960 340 A1**
(43) Date of publication of application: **30.12.2015**
(21) Application number: 13862657.7
(22) Date of filing: 11.12.2013
(51) Int. Cl.: C12Q 1/68

(54) **METHOD FOR INFLUENCING PROLIFERATIVE STATUS OF CELLS USING SPECIFIC G-CHAIN NUCLEOTIDE SEQUENCES OF HUMAN TELOMERIC DNA**

(30) Priority: 12.12.2012 RU 2012153541
(71) Applicant: Chiryasova, Elena Andreyevna, Leninsky raion, Moskovskaya oblast 142714 (RU)
(72) Inventor: Chiryasova, Elena Andreyevna, Leninsky raion, Moskovskaya oblast 142714 (RU)
(74) Representative: Spengler, Robert
(86) International application number: PCT/RU2013/001108
(87) International publication number: WO 2014/092609

(57) **Abstract**

The invention relates to the field of molecular and cell biology. Proposed is a method for influencing the proliferative status of cells using specific G-chain oligonucleotide sequences of human telomeric DNA, said sequences imitating open forms of telomeric overhangs in cells, where, unlike the traditional concept of the cytostatic use thereof, they are seen not as signals of DNA damage, but rather as molecular and quantum-optical signals regulating the transition between phases of the cell cycle. The invention allows for significantly increasing the specificity and effectiveness of telomere DNA-therapy by means of using specific nucleotide sequences on the basis of six variations of triplet endings (SEQ ID NO: 7-12). The ending SEQ ID NO: 8 comprises the basis for the effect which suppresses division, and the ending SEQ ID NO: 12 is at the foundation of stimulating cell proliferation. The effectiveness of the influence of the endings is regulated by selecting the length and initial nucleotides of the sequences, which determine the base variations of telomeric repetition (SEQ ID NO: 1-6). Forming corrective mixtures of triplet endings of the telomere oligonucleotides on the basis of an initial determination of the terminal nucleotides of the telomere DNA in cells allows for compensating for insufficient endings at specific stages of the cell cycle. For malignant cells, this provides a normal progression and duration of division. The invention is intended for influencing the proliferative status of cells, with the goal of suppressing or stimulating division, and also for controlling the time at which certain phases of the cell cycle come about, and can be used in medicine for treating oncological diseases, in immunology for controlling the body's immune status, and in transplantology, gerontology and cosmetology for maintaining the ability of cells to regenerate and rejuvenate the body.

## Description

### The abstract

The invention relates to the field of molecular and cell biology. At the proposed method the specific oligonucleotide sequences of G-strand of human telomeric DNA, imitating open form of telomeric overhangs in cells, are considered not as sig28nals of DNA damage, unlike the traditional concept of their cytostatic use, but rather as the molecular and quantum-optical signals, regulating gene expression and transition between phases of the cell cycle. The invention allows to significantly raise the specificity and efficiency of telomeric DNA-therapy by means of using specific nucleotide sequences on the basis of six variants of their triplet endings (SEQ ID NO: 7-12). Thus, the ending SEQ ID NO: 8 composes a basis of influence, depressing cell division, and the ending SEQ ID NO: 12 underlies a stimulation of the cell proliferation. The efficiency of influence of the telomeric endings is controlled by selection of length and initial nucleotides of the sequences, which define the basic variants of human telomeric repeat (SEQ ID NO: 1-6). The composition of correcting mixtures of the triplet endings of telomeric oligonucleotides on the basis of preliminary definition of the Terminal Nucleotide Profiles of telomeric DNA in cells allows compensating the missing endings at certain moment of the time for current stages of the cell cycle. For malignant cancer cells it provides a normal progression and duration of division. The invention is designed for influence on the cell proliferative status for the purpose of suppression or stimulation of cell division, and also for controlling of time of the coming of certain phases of cell cycle. It can be used in medicine for treatment of oncological diseases, in immunology for controlling of the immune status of human organism, in transplantology, gerontology and cosmetology for maintaining the ability of cells to regeneration and organism rejuvenation.

### Description

### FIELD OF THE INVENTION

The invention relates to the field of molecular genetics and cellular biology and is designed for influence on cell division by means of the single-stranded termini of human telomeric DNA (G-strand overhangs). The declared method is applied to making of correcting influence on the cell proliferative status for the purposes of cell division suppression or activation. The correction is based on the specific influence of various nucleotide sequences of human telomeric repeats and their triplet nucleotide endings on progression of the cell cycle phases due to the regulation of gene expression controlling it. The use of specific nucleotide endings of telomeric G-overhangs is responsible for time control of the coming of cell cycle phases, regulates the duration of cell cycle and leads to suppression or stimulation of cell division. It opens new prospects for application of synthetic analogues of telomeric DNA-sequences for regulation of the proliferative status of different cells and tissues. The invention is new type of DNA-therapy and can applicate in medicine in treatment of oncologic diseases, in immunology for monitoring of the immune status of human organism, in gerontology, dermatology and cosmetology for maintenance of ability of cells and tissues to regeneration and organism rejuvenation.

### BACKGROUND OF THE INVENTION

It is known that treating of cells by synthetic analogues of single-strand telomeric overhangs (T-oligos) invokes development in them of the processes similar to DNA damage responses. Thus, the oligonucleotides, which have been not relate to telomeric sequences, are not awake, and synthetic analogues of telomeric G-strand are awake and play a role opened (not formed a loop) forms of native overhangs. Their overabundance is signal of DNA damage, which starts the processes of reparation and arresting of cell cycle. The traditional concept of using synthetic analogues of telomeric overhangs is based on this principle for cell growth inhibition.

It has been revealed, that the synthetic telomeric sequences quickly collect in a cell core and activate a series of regulatory proteins (ATM, p53, r95/Nbs1, p16, pRb, E2F1), that is a basis of anticancer therapy. Thus, T-oligos invoke the inhibition of cancer cells various paths. Their influence has led to development of apoptosis with stabilization in sub-G₀-G₁ phases for skin melanoma cells [Puri N., Eller M.S., Byers H.R. Dykstra S., Kubera J., Gilchrest B.A. Telomere-based DNA damage responses: a new approach to melanoma (2004) FASEB J. Vol. 18, Nº12, pp. 1373-1381], breast carcinomas [Yaar M., Eller M.S., I., Kubera J., Wee L.H., Cowan K.H., Gilchrest B.A. Telomeric DNA induces apoptosis and senescence of human breast carcinoma cells. Breast Cancer Research (2007) Vol.9, No.1, 13 pages, PMID: 17257427], ovary cells [Sarkar S., Faller D.V. T-oligos inhibit grows and induce apoptosis in human ovarian cancer cells. Oligonucleotides (2011) Vol.21, No.1, pp. 47-53]. In glioma cells the T-oligos invoked autophagy, not related to apoptosis through inhibition of transcriptional factor STAT3 and autophagy regulator mTOR [Aoki H., Iwado E., Eller M.S., Kondo Y., Fujiwara K., Li G.Z., Hess K.R., Siwak D.R., Sawaya R., Mills G.B., Gilchrest B.A., Kondo S. Telomere 3 '-overhang-specific DNA oligonucleotides induce autophagy in malignant glioma cells. FACEB J. (2007) Vol. 21, No. 11, pp. 2918-2930].

In normal fibroblasts the synthetic analogues of G-overhangs invoked cell cycle arrest in a synthetic phase and development a senescent phenotype, related to activation p53 p21, p16, and pRb pathways [Eller M.S., X., Liu S., Hanna K., Backvall H., Opresko P.L., Bohr V.A., Gilchrest B.A. A role for WRN in telomere-based DNA damage responses. PNAS (2006) Vol.103, No. 41, pp. 15073-15078].

Thus, some examples of the T-oligos, using in scientific literature, are the close prototypes of the declared invention. It is important that more often were used 11-base oligonucleotide pGTTAGGGTTAG, ended in terminal triplet TAG, as a basis of anticancer therapy [Puri N. et al., 2004; Eller M.S. et al., 2006; Aoki H. et al., 2007; Yaar M. et al., 2007]. Other variants of the nucleotide endings, for example, dimer pTT and 16-mer oligonucleotide pGTTAGGGTTAGGGTTA have not shown the expressed influence on cell division inhibition. However, the treatment of melanoma cells using 16-base oligonucleotide with TTA ending has invoked the angiogenesis inhibition [Coleman C., Levine D., Kishore R., Qin G., Thorne T., Lambers E., Sasi S.P., Yaar M., Gilchrest B.A., Goukassian D.A. Inhibition of melanoma angiogenesis by telomere homolog oligonucleotides. J. Oncology (2010), 14 pages, PMID: 20652008].

The dimer pTT, which has very low degree of homology with telomeric DNA due to the very short length, has invoked the intensifying of hair follicle pigmentation [Atoyan R.Y., Sharov A.A., Eller M.S., Sargsyan A., Botchkarev V.A., Gilchrest B.A. Oligonucleotide treatment increases eumelanogenesis, hair pigmentation and melanocortin-1 receptor expression in the hair follicle. Experimental Dermatology (2007) Viol.1 6, No.8, pp. 671-677].

The important results have been received at comparison the hydrolyzable phosphodiester-linked form and nonhydrolyzable phosphorothioate-linked (PS) form of 11-base T-oligos with ending TAG [Eller M.S., X., Liu S., Hanna K., Backvall H., Opresko P.L., Bohr V.A., Gilchrest B.A. A role for WRN in telomere-based DNA damage responses. PNAS (2006). V.103, pp.15073-15078]. Only phosphodiester-linked form, which completely corresponded to physiological conditions of telomeric G-overhangs existence, has invoked an apoptosis in human melanoma cells within 4 days with stabilization in sub-G₀-G₁ phases. Not capable to hydrolysis PS-T-oligos with blocked 3'-end were not efficient. At normal cells the treatment using completely hydrolyzable T-oligos has led to accumulation of cells within the S-phase of cell cycle. The half-life studying of telomeric oligonucleotides has shown that PS-T-oligos had much longer half-life, than not blocked form. It testified that not blocked form is a substrate for cleavage by nucleases unlike phosphorothioate-linked form. Hereby, the blocking of 5'-end with PS linkages did not matter.

Thus, necessity of the open 3'-end of telomeric G-overhangs, which assumes the possibility of its hydrolysis by exonucleases and the appearance of new endings distinct from TAG has been shown. Despite it, there are no references, investigating the influence on the cell division progression and the coming of cell cycle phases by specific telomeric sequences, differing in telomeric repeat variations and all possible versions of its nucleotide endings. On this distinguishing character of telomeric sequences for the declared invention there is no direct analogue.

Earlier T-oligos were used only as the molecular signals, testifying to disruptions of the telomere loop structure, and leads to cell cycle arrest and induce apoptosis. Thus, it was not given any attention of the specific sequence of telomeric oligonucleotides, as genetic regulating information. More often was applied TAG ending of the telomeric overhangs, which was added to culture medium in final concentration 40 mkM, that considerably exceeded its physiological quantities in cells. Such concentration excess led to toxic-like inhibiting effects for cells, relating to supernormal DNA-damage signaling and cell cycle arrest. In some cases it led to apoptosis and autophagy of cells and compounded the old concept of using synthetic analogues of telomeric overhangs for influence on the cell proliferation. Thus, more often T-oligos were applied in anticancer therapy as agents, suppressing cell division and inhibiting cell growth. It never used as agents, controlling proliferative status and time of the coming of cell cycle phases, or as stimulants of cell proliferation and as factors, shortening or lengthening of the cell cycle.

### DISCLOSING OF THE INVENTION

The main technical task of the declared invention is working out of a method for using of telomeric sequences within physiologically non-inhibiting concentration, based on understanding of differences of their nucleotide sequences and endings, as genetical information, regulating cell division. In this connection the 100% homology to G-strand sequence of human telomeric DNA is distinguishing characteristic of the declared method. The technical result of the invention is the decrease of toxic-like effect, relating to DNA-damage signaling and inhibiting the cell growth. The other technical result is the rising of influence specificity of the synthetic analogues of telomeric overhangs, for changing of cell proliferative status and achievements of various therapeutic effects, relating to control of the coming of cell cycle phases.

The essence of the invention consists in use of water solutions of specific nucleotide sequences of the G-strand of human telomeric DNA in final concentration, less than 40 mkM in the cell medium. Thus, there is a free infiltration of telomeric sequences into cells, where they carry out a role of the signals, supervising progression of cell cycle phases, and carry out regulation of the proliferative status of cells for the purpose of stimulation or suppression of their division.

The principles of use of the declared invention are based on the revealing of terminal nucleotide polymorphism of the G-strand telomeric overhangs, depending on the time and the cell cycle phases. The telomeric DNA is dynamical system, in which is carried out the continuous synchronized process of changes of its triplet nucleotide endings, compounding the terminal nucleotide transitions cycle. Thus, the efficiency of T-oligos treatment will depend on, what moment of this cycle the T-oligos introduction will be made, because they must correspond to the current native Terminal Nucleotide Profiles in cells-recipients. In this connection the examination of Terminal Nucleotide Profiling of telomeric DNA is very expedient.

For the division activating state of cells is characteristic the unbalanced nonequilibrium profile of G-strand terminal nucleotides of telomeric DNA, which relates to the trigger moment of transferring Go phase to G1 phase of the cell cycle. It is connected with the sharp long increase of quantity of the TTA endings of telomeric overhangs (till 70% and more). Thus, percentage of terminal triplets TAG, AGG and other reduces to zero. For the nondividing state of cells in G₀ phase, and also for the state of cells activated to division, after 10-12 hours from the beginning of stimulant influence, is characteristic the balanced equilibrium profile of G-strand terminal nucleotides. For it characteristic of high content of the terminal triplet AGG approximately equal the quantity of the triplet GTT (25-27 %). Thus, quantity of ending TTA approximately equal the quantity of the ending TAG (16-20 %), and terminal triplet GGT approximately equal the quantity of the triplet GGG (5-7 %).

The existence conditions of the described profiles are provided with randomization mechanisms of the 3'-end of telomeric overhangs due to the removal of terminal nucleotides by exonuclease. The cycle of consecutive single nucleotide transitions on the 3'-end of telomeric DNA leads to formation of following terminal triplets: AGG→TAG→TTA→GTT→GGT→ GGG→AGG.

Application of the studying of fluorescent properties and spectral characteristics of DNA nucleotide sequences, by means of the quantum-bound radiation spectrum of dyes with free fluorophoric groups, has allowed revealing the specific spectral characteristics of telomeric repeat variations, in which telomeric G-overhangs can terminate. They lay down a basis of spectral coding of the proliferative information in cells and define the colour markers of resultant fluorescence of telomeric overhangs, which characteristic for the nondividing and activated to division states of cells. For nondividing state of cells, in which the presence of terminal triplets AGG and TAG is characteristic, the growth of green fluorescence is observed. At division activation state of cells the fluorescence in red spectrum is prevails and relates to growth of TTA ending content of telomeric overhangs. Thus, the modern conception about a role of nucleotide sequence of the telomeric repeats and their terminal endings, which composed the telomeric overhangs, is based on their understanding as substrates of the light energy, controlling the cell division.

The mechanism, which combines telomeric repeat variations, its various triplet endings and different length, provides the existence in cells of many kinds of changeable telomeric overhangs. Each of them has specific characteristics and unique action in cells, and composes the basis of genetic biocomputer, which regulates gene expression. In this connection a new conception of using of telomeric G-strand overhangs and its synthetic analogues has been formulated and has laid down in a basis of the declared method. Each telomeric overhang in the cells is presented by one of six variants of the telomeric repeat sequences, presented in SEQ ID NO: 1-6, which terminates in a specific triplet ending of the terminal nucleotides. At the ending of telomeric overhang by the not full-length repeat, its terminal triplet depends on its length. In this connection the special role is got by a terminal triplet of nucleotides, in which telomeric overhangs terminate. The each terminal triplet can be one of the variants, presented in SEQ ID NO: 7-12.

The terminal triplet TAG, which was applied earlier to influence on the dividing cells, represents only one of variants of the telomeric overhang endings, arising in a cycle of terminal nucleotide transitions: AGG→TAG→TTA→GTT→GGT→GGG→AGG. Its depressing division function widely used in anticancer therapy, is based that it is one of terminal triplets, characteristic for nondividing state of cells. It has been shown, that the terminal triplet TAG is absent at the malignant lymphocytes in mitosis phase, at which it certainly should be present as the criterion of normal progression of cell division. Reduction or total absence of AGG and TAG terminal triplets in all phases of cell cycle except for the moment of transferring from Go to early G₁ phase can be the criterion of cell malignization.

In this connection the AGG (SEQ ID NO: 8) variant of the telomere endings specific for nondividing state of the cells, specifically for periodic balanced profile of the DNA terminal nucleotides and for tissue-specific program of the cell development. It forms an influence basis on the cell proliferative status for the purpose of division suppression and switching-off the embryo-specific program of their development. It causes its more efficient use in anticancer therapy in comparison with TAG variant of the telomeric DNA endings.

The variant of the telomeric overhang endings with TTA (SEQ ID NO:12) terminal triplet in physiologically adapted concentration, nontoxic and not leading to arrest of the cell cycle, can form a basis of the influence stimulating cell division. Efficacy of its use is defined by its connection with the forming of unbalanced profile of the G-strand terminal nucleotide of telomeric DNA at cell division activation. This terminal nucleotide profile is connected to activation of embryo-specific program, suppression of tissue-specific program of cell development and occurrence of nonequilibrium quantum state of cells with prevalence of red fluorescence of telomeric overhangs.

Thus, the newest concept of telomeric overhangs application for correction of the cell proliferative status recognizes, that each nucleotide sequence variant of human telomeric repeat and its terminal nucleotide triplet plays a role of molecular and quantum signal. The interrelation of these signals is very important for cell division regulation. In this connection the basis of declared influence on the cell proliferative status is compounded by variants of the nucleotide sequences of telomeric overhangs and especially their endings. Thus, not only the single oligonucleotide sequences can be used, but also the mixtures with a fixed quantitative interrelation of various telomeric sequences.

At the making of correcting mixtures of synthetic telomeric oligonucleotides it is recommended to carry out the preliminary terminal nucleotide profiling of telomeric DNA for cells-recipients in anticancer therapy. It allows to determinate the optimum ratio of terminal triplets TAG:AGG:GGG (SEQ ID NO:7,8,9) in correcting mixture, which depends on their native concentration in cells at the certain moment of the time.

The one of the possible directions of the declared method is application of the modified synthetic analogues of telomeric overhangs. Modification can consist in introduction of phosphorothioate-linkage groups (P=S), preventing hydrolysis and eliminating of concrete terminal nucleotides. For example, the introduction of such linkages in sequence of repeats GsGsG (SEQ ID NO:9), A_{S}G_{S}G (SEQ ID NO:8) and TA_{S}G (SEQ ID NO:7) can prevent realization of the terminal nucleotide transitions GGG→AGG→TAG→TTA. Thus, in a cycle of 3'-end single nucleotide transitions of telomeric DNA will again not arise the terminal triplet TTA activating division that is very important for anticancer therapy. Introduction of phosphorothioate-linkage groups in sequence of terminal triplet TsTsA (SEQ ID NO:12) will protect it from transformation in terminal nucleotide transitions cycle and will conserve its stimulating effect.

The second modification direction of synthetic telomeric overhangs is use of fluorescent labels and quenchers. It allows enhancing or reducing the natural fluorescence of synthetic analogues of telomeric overhangs in concrete spectrum. For cell division suppression and activation of tissue-specific development program use of fluorescent labelling of AGG-terminated oligonucleotide by green fluorescent dyes in a wave range of 520-540 nanometers. For example, the carboxyfluorescein dye is perspective for this purpose. Thus, it is jointly possible to use quenchers of red fluorescence similar to RTQ-2 or BHQ-3.

For cell division activation and starting of the embryo-specific development program is perspective the modification of the TTA-terminated oligonucleotide with combination of fluorescent dyes in orange-red and red spectrums (ROX, Cy3.5, Cy5, Cy5.5, Texas Red) and quenchers of green spectrum (RTQ1, BHQ1). Similar to a green spectrum for the development of photoreactions in cells has a dark blue spectrum. In this case the use of fluorescent dyes and quenchers of dark blue spectrum is possible.

It is necessary to notice that application of fluorescent dyes widely used for DNA labelling can be partly limited due to big quantum yield not corresponding to physiological level of photoreactions in cells. It is known that cells have a certain limit of photonic saturation, above which to reach the positive reactions, for example, to stimulate the cell division by mitogenetic radiation is not possible. In this connection the designing of special fluorophores with suitable quantum yield, including the natural fluorescent metabolites of cells is perspective.

The third modification direction of synthetic telomeric overhangs is use of photosplitting linkages, which can provide the destruction of telomeric oligonucleotides, controlled in time under the influence of certain length of light waves. Introduction of such linkages allows regulating eliminating of terminal nucleotides and providing the controllable modifications of terminal triplets, according to cycle of 3'-end single nucleotide transitions of telomeric DNA endings during the cell cycle.

One of variants of the declared method realization is use of native fractions of telomeric overhangs. The characteristic feature of this variant is that the native fraction of telomeric overhangs represents heterogeneous polynucleotides with different length and nucleotide endings physiologically adapted for performance of functions at certain stages of the cell cycle. Actually this variant of invention realization represents the mechanism of transfer of the proliferative information from one cell to other cells by means of DNA molecular substrate. For these purposes are used the cell donor cultures, which synchronize at the specific stages of cell cycle for extraction of the native telomeric overhang fractions. Difficulty in use of these fractions consists in impossibility of avoidance of their incorporation in terminal nucleotide transitions cycle due to the exonuclease influences in cells-recipients. In this connection the effect of their influence can be more short-term, than at use of the modified synthetic analogues of telomeric overhangs with the protecting groups preventing their hydrolysis.

Cell division regulation is realized by the proliferative information, written down in sequence variations of DNA telomeric repeats and controlled by their nucleotide endings. The regulation mechanism is carried out at the level of gene expression by principle of long-range interaction and superposition of different soliton waves, including optical. The single-strand telomeric overhangs, which nucleotides have the maximum degree of freedom in comparison with double-stranded DNA, play role like ideal polygon for formation the rotationally oscillatory movements of soliton waves. It is revealed that the soliton dynamics is sensitive to a point of initiation and depends on the nucleotide sequence of DNA. Reflecting the specific nucleotide sequence of telomeric repeats, dynamics of soliton disturbance is the referent of reading of the proliferative information and is capable to carry out the long-range influence on gene expression, which regulate cell division. Thus, the specific fluorophor-bound fluorescence of telomeric overhangs can be a necessary source of photonic pump for optical soliton formation.

Within development of wave and bioresonant medicine probably use of various wave equivalents (replicas) of telomeric overhangs forming the correcting wave patterns on the basis of declared nucleotide sequences and endings. Similar equivalents in the form of electromagnetic waves, sound or torsion vibrations and other fields of vacuum and quantum nature, including holographic or soliton origins, can be used for the purpose of rejuvenation and prolongation of life of the human organism, for correcting of its proliferative and immune status, treatment of cancer and other diseases. The directed influence of declared telomeric sequences on the proliferative status of cells-recipients in the form of the wave information lies at the basis of invention. It defines the immediate prospects of use of the declared sequences of telomeric overhangs in the field of biology, biotechnology and medicine.

### SEQUENCE LISTING FREE TEXT

The cell division regulation is carried out by the proliferative information written down in variations of hexanucleotide repeat sequences of the G-rich strand of telomeric DNA and controlled by their nucleotide endings. The declared method allows to compose the specific nucleotide sequences of human telomeric DNA on the basis of six basic variants of the telomeric repeat sequences, presented in SEQ ID NO: 1-6: GGTTAG, GTTAGG, TTAGGG, TAGGGT, AGGGTT, GGGTTA.

The sequences can consist of n-th number of one of the basic sequences, presented in SEQ ID NO: 1-6, and terminate in full-length or not full-length telomeric repeat. Thus, the special role for influence on the cell proliferative status has the specific combination of terminal nucleotides of the telomeric sequences, compounding their terminal triplet from among TAG, AGG, GGG, GGT, GTT and TTA (SEQ ID NO: 7-12).

### BRIEF DESCRIPTION OF THE DRAWINGS

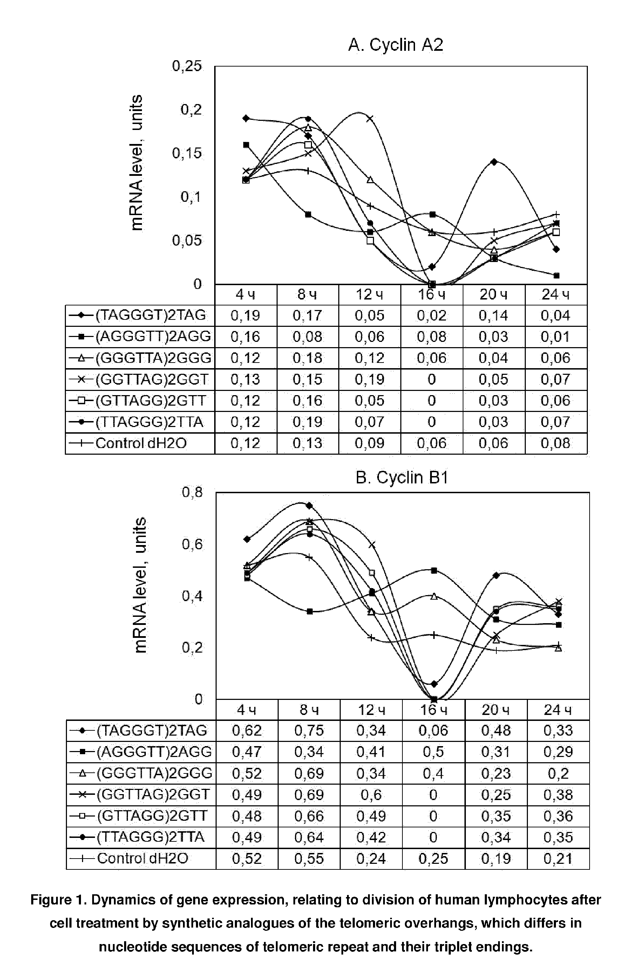

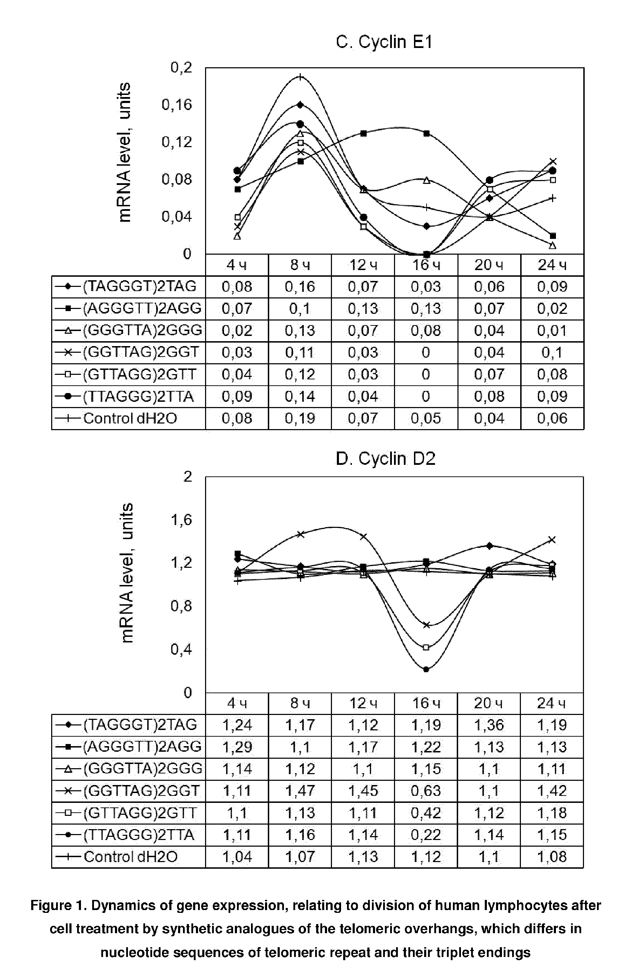

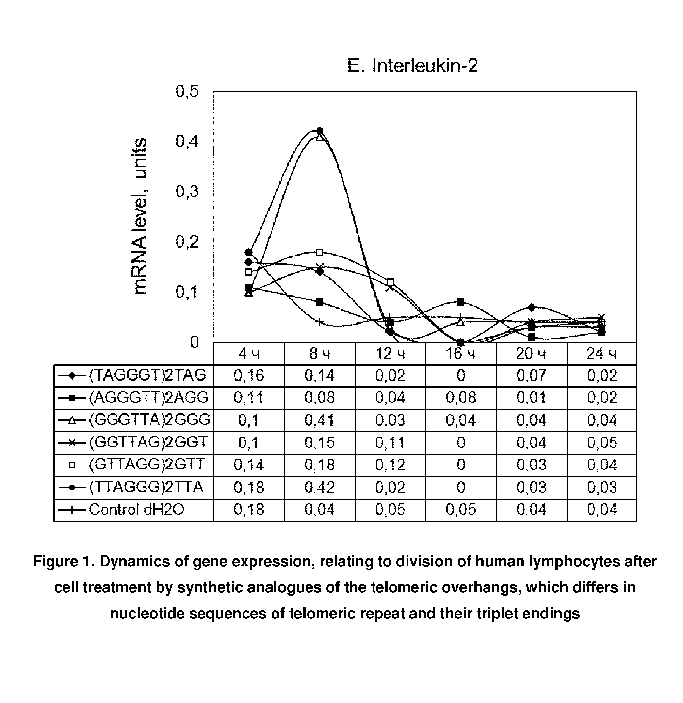

**Figure 1****.** Dynamics of gene expression, relating to division of human lymphocytes after cell treatment by synthetic analogues of the telomeric overhangs, which differs in nucleotide sequences of telomeric repeat and their triplet endings (for example the final oligonucleotide concentration 20 mkM). It displays dynamics of changes of the gene expression for 24 hours at intervals of 4 hours. (A) - mRNA levels of cyclin A2; (B) - mRNA levels of cyclin B1; (C) - mRNA levels of cyclin E1; (D) - mRNA levels of cyclin D2; (E) - mRNA levels of interleukin-2.

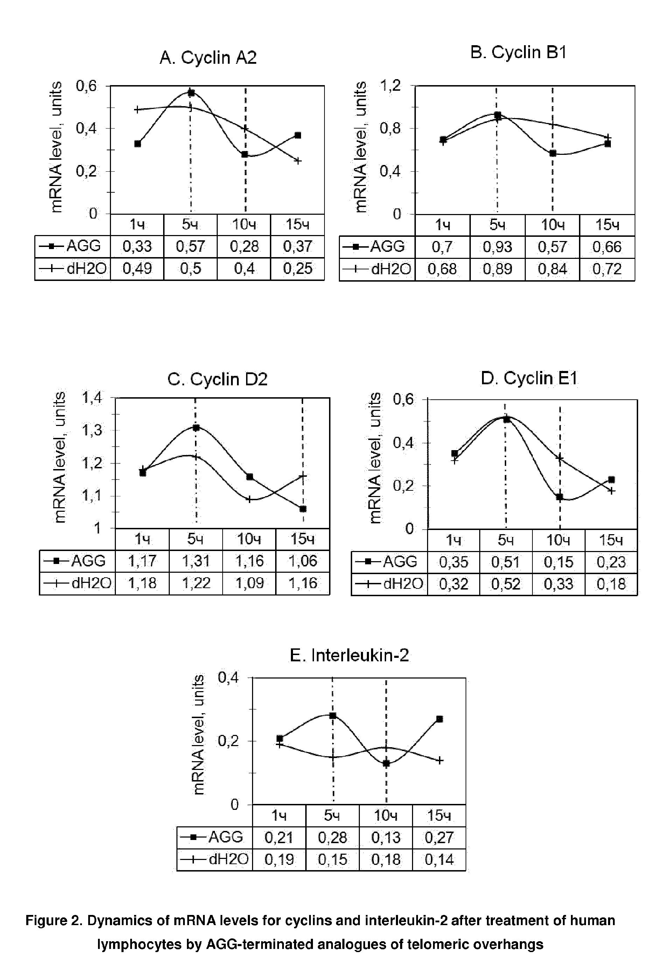

**Figure 2****.** Dynamics of mRNA levels for cyclins and interleukin-2 after treatment of human lymphocytes by AGG-terminated analogues of telomeric overhangs (for example (AGGGTT)₂AGG oligonucleotide in final concentration 10 mkM). It displays dynamics of changes of the gene expression for 15 hours at intervals of 5 hours. The level maximums of gene expression are noted by dash-dot line, and level minimums - by dashed line.

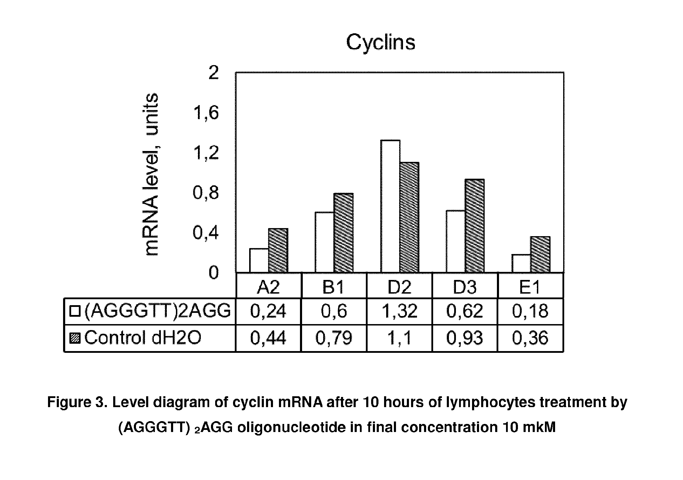

**Figure 3****.** Level diagram of cyclin mRNA after 10 hours of lymphocytes treatment by (AGGGTT) ₂AGG oligonucleotide in final concentration 10 mkM.

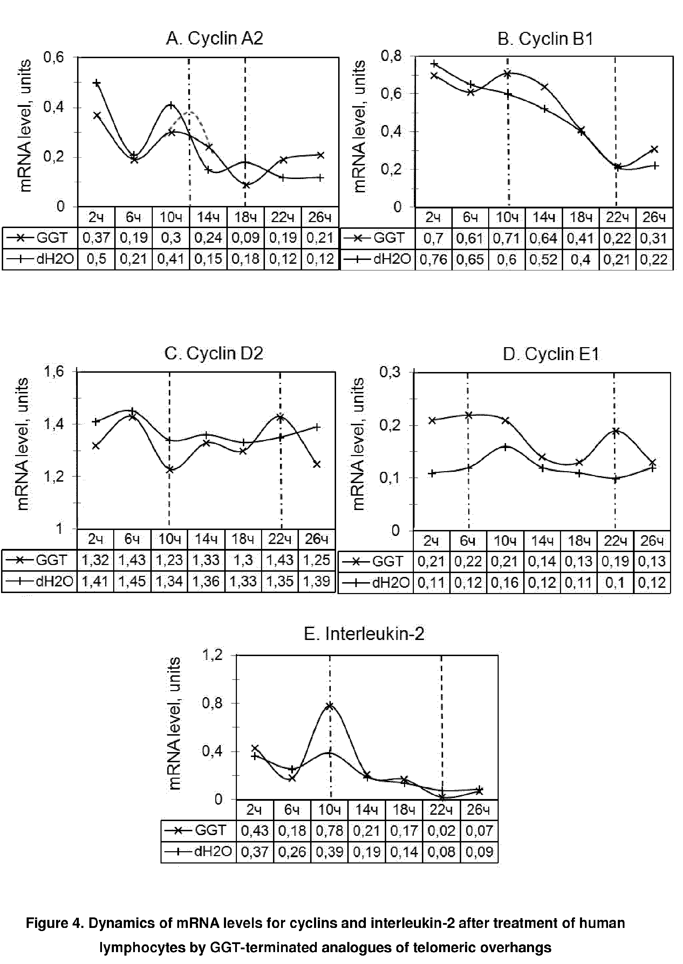

**Figure 4****.** Dynamics of mRNA levels for cyclins and interleukin-2 after treatment of human lymphocytes by GGT-terminated analogues of telomeric overhangs (for example (GGTTAG)₂GGT oligonucleotide in final concentration 10 mkM). Displays dynamics of changes of the gene expression for 26 hours at intervals of 4 hours. The level maximums of gene expression are noted by dash-dot line, and level minimums - by dashed line.

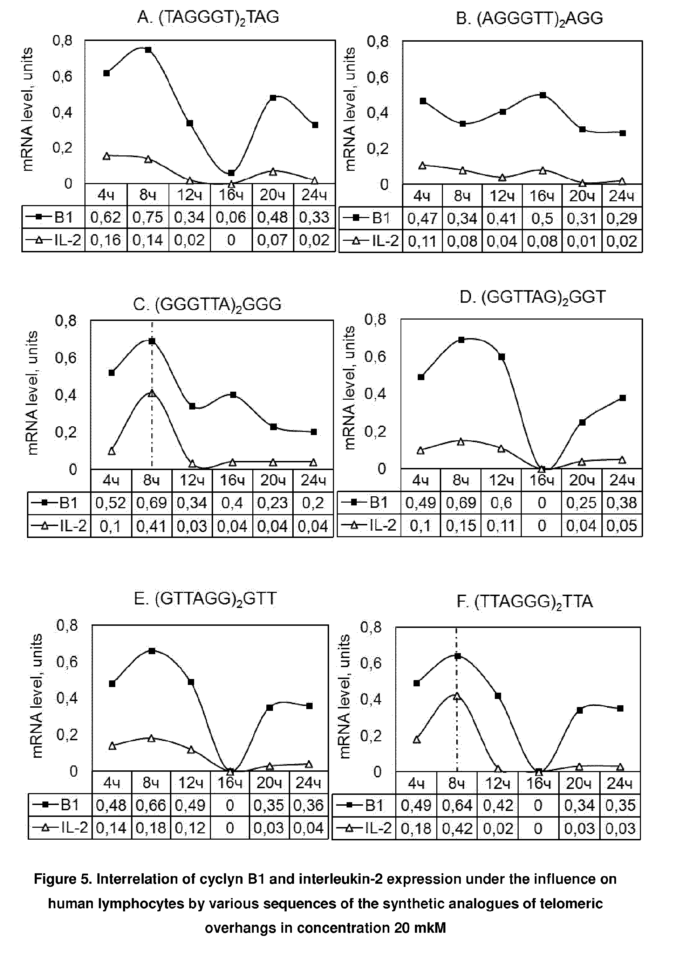

**Figure 5****.** Interrelation of cyclin B1 and interleukin-2 expression under the influence on human lymphocytes by various sequences of the synthetic analogues of telomeric overhangs in concentration 20 mkM. The associated maximums of gene expression are noted by dash-dot line.

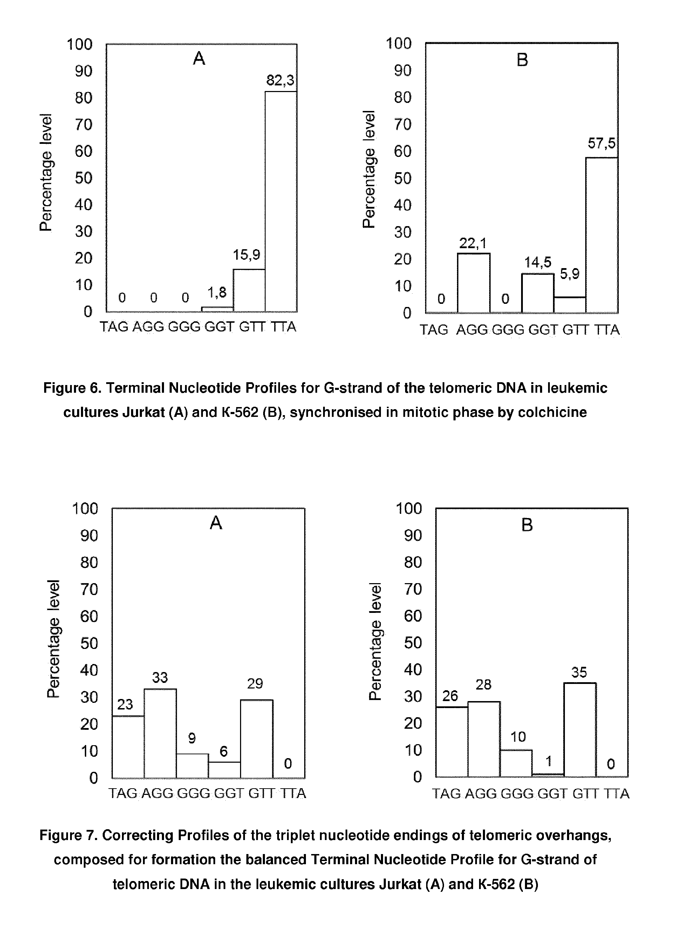

**Figure 6****.** Terminal Nucleotide Profiles for G-strand of the telomeric DNA in leukemic cultures Jurkat (A) and K-562 (B), synchronized in mitotic phase by colchicine.
**Figure 7****.** Correcting Profiles of the triplet nucleotide endings of telomeric overhangs, composed for formation the balanced Terminal Nucleotide Profile for G-strand of telomeric DNA in the leukemic cultures Jurkat (A) and K-562 (B).

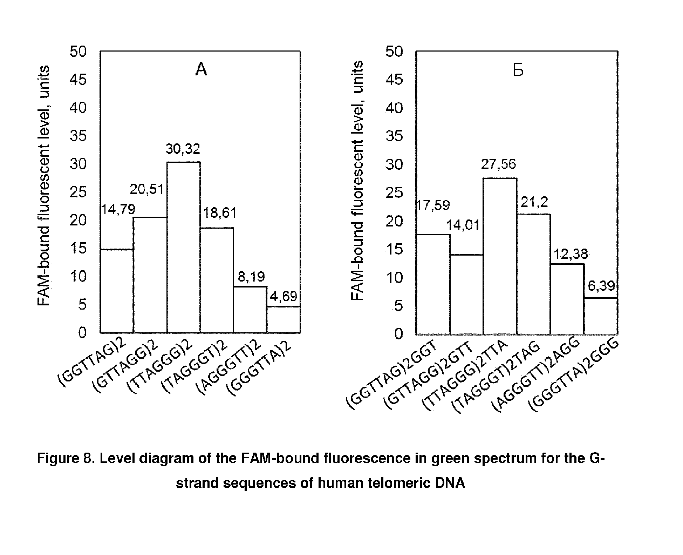

**Figure 8****.** Level diagram of the FAM-bound fluorescence in green spectrum for the G-strand sequences of human telomeric DNA. (A) - variants of the telomeric sequences, consisting from two full-length hexanucleotide repeats. The sequence formula: 2n, where n - the telomeric repeat, presented in SEQ ID NO:1-6; (B) - variants of the telomeric sequences, consisting from two full-length hexanucleotide repeats, terminating in not full-length repeat, in the terminal triplet, presented in SEQ ID NO: 7-12. The sequence formula: 2n+1/2n, where n - the telomeric repeat, presented in SEQ ID NO: 1-6.

### EXAMPLES

The declared invention is illustrated by examples, showing the possibility of variations of the G-strand sequences of human telomeric DNA for influence on the cell proliferative status. The human peripheral blood lymphocytes were taken as basic model for studying of their influence. For control of cell proliferative status the research of expression of the marker genes regulating cell division was used. The specific expression of cyclins A, B, E and D, which characteristic for certain phase of cell cycle is shown for lymphocytes stimulated to division. In this connection the genes of cyclins A2, B1, E1 and D2 and also interleukin-2 (IL-2), linked to cell proliferation, have been chosen for studying of influence of the specific sequences of telomeric overhangs on the cell cycle of human lymphocytes.

The direct dependence of cyclin expression from sequence variation of the telomeric repeat and the triplet endings of telomeric sequences specifies their indisputable influence on the progression of cell cycle phases.

### EXAMPLE 1. Influence on the proliferative status of human lymphocytes by telomeric DNA sequences, differing in variations of the nucleotide sequence of G-strand repeat and the triplet endings.

The peripheral blood lymphocytes of human donor, which been ill by influenza infection, were taken for obtaining of cell culture. These cells were characterized by natural activation of division within two weeks of the investigated period.

The synthetic oligonucleotides, synthesized by standard method of synthesis in company "Evrogen" (Russia), were used for influence on the cell culture. Oligonucleotides were representing the sequences of G-strand of human telomeric DNA, imitating its single-strand overhangs by length 15 nt:
1. (TAGGGT)₂ TAG;
2. (AGGGTT)₂ AGG;
3. (GGGTTA)₂ GGG;
4. (GGTTAG)₂ GGT;
5. (GTTAGG)₂ GTT;
6. (TTAGGG)₂ TTA.

Each sequence consisted of variant of two full-length telomeric repeats, presented in SEQ ID NO: 1-6, and terminated in six possible variants of terminal triplet among SEQ ID NO:7-12.

The sterile water solutions of oligonucleotides were added to culture medium RPMI-1640 in final concentration 20 mkM. The part of cell culture, in which the oligonucleotide solution has been substituted by sterile distilled water, was used as control sample. The moment of the time, corresponding to most balanced Terminal Nucleotide Profile of native telomeric DNA, which characterized by most close percentages of the triplet endings in ratio: TAG (16,4%); AGG (13,1%); GGG (16,2 %); GGT (10,1%); GTT (15,4%); TTA (28,8%), was chose for oligonucletide introduction. Close percentages allow assessing the role each of endings at close degree.

For assessment of telomeric oligonucleotide influence on the proliferative status of cells carried out the consecutive cell sampling during 24 hours at intervals of 4 hours. The obtained cells used for extraction of total RNA and statement of standard procedure RT-PCR. For the analysis of gene expression the densitometric analysis of electrophoretic separation in agarose gel of amplification products of mRNA sequences of investigated genes in the presence of ethidium bromide was used. The expression levels of each gene was defined, as the quantitative ratio of its mRNA level to mRNA level of normalizing genes GAPDH and ACTB, estimated as unit of mRNA level.

The following curves of changes of mRNA levels for cyclins and interleukin-2 under the influence of telomeric sequences have been received (Fig. 1). The curves testify that used variants of telomeric oligonucleotides have invoked the circular changes of expression of the genes, related to cell division. Thus, each variant of telomeric sequences led to specific changes in dynamics of cyclins and interleukin-2. Further at the detailed consideration of each sequence action we will focus the attention on kind of the triplet nucleotide endings. We will mean, that specific action is shown by whole telomeric sequence, including unique variation of nucleotide repeat and not just a terminal triplet.

The greatest changes of cyclin dynamics were caused by the second variant of sequence presented by two telomeric repeats AGGGTT (SEQ ID NO: 5) with terminal triplet AGG (SEQ ID NO:8). The ending AGG sharply differed on its action from the others and was the original antagonist of all other variants of the endings of telomeric overhang. Character of its influence on dynamics of the basic cyclins was in an antiphase in comparison with all other variants of the endings, including control sample. In intervals where the increase of cyclin expression under the influence of other variants of terminal triplets was observed (the convex form of the curves), in the sample treated by AGG variant of the ending was observed the decrease of cyclin mRNA levels (the concave down form of the curves). And, on the contrary, growth of gene expression following this stage under the influence of AGG ending was accompanied by falling of gene expression in other samples. Such wavy character of cyclin dynamics, relates to antiphase influence of AGG variant of the telomeric endings, was especially clearly displayed for cyclins A2, B1 and E1 (Fig. 1 A-C).

Cyclin E1 under the influence of AGG-terminated telomeric sequence was characterized by late increase of expression (Fig. 1 C). Thus, while its mRNA level started to fall under the influence of other variants of the endings, the sample keeping AGG-terminated oligonucleotides has revealed the proof growth of mRNA level with peak at 12-16 hours. It is known that the expression of cyclin E1 provides progression of G₁ phase to early synthetic S phase and occurs after 18 hours from the beginning of stimulation of lymphocytes to division by PHA.

This fact is important for understanding of a course of cell division as periodic increase of AGG ending with decreasing of TTA ending of telomeric overhangs is characteristic for nondividing state of cells. Gradual destruction of AGG endings in cells and its transformation into other endings due to the serial exonuclease effects, leads to putting off its suppressing action and consecutive transition of cells to G₁ phase and then to the synthetic phase. It is accompanied with expression increase of cyclin E1 during 16-18 hours of oligonucleotide treatment. In this connection there is clear a role of AGG ending in definition of time of the coming of synthetic phase of preparation for cell division.

In comparison with other endings, the AGG variant had the general suppressing effect on the interleukin-2 expression, displayed at first 12 hours (Figs. 1 E). However, the different experiment with use of other time intervals has revealed a maximum of interleukin-2 expression in 5 hours an example 2 (Fig. 2 E). Similar expression maximums were observed also in 8 hours for TTA and GGG endings and in 10 hours for terminal triplet GGT.

The AGG-terminated analogue of telomeric overhang has not shown strongly pronounced influence on the expression of cyclin D2 (Fig.1 D). Probably, it is connected with that the cells in Go phase of cell cycle, when the expression of cyclin D2 is possible, are characterized by often periodic prevalence of AGG variant above TTA variant of telomeric endings (the basic state of nondividng cells in a resting phase). It is known that cyclin D2 expresses in Go and early G₁ phases of lymphocyte cell cycle and cyclin D3 is absent in G₀ phase and increases in late G₁ phase. In this connection we have assumed, that AGG ending will make stronger influence on expression of cyclin D3, which uniquely testifies to cell advance to G₁ phase of preparation for division. The additional experiment has been made in support of the general character of AGG-terminated oligonucleotides, relating to its suppressing action on expression of major cyclins at the first 10-12 hours. In this experiment the influence of sequence (AGGGTT) ₂AGG on the mRNA levels of cyclins A2, B1, E1, D2, including cyclin D3 was studied an example 2.

For 4 hours of influence of the telomeric sequence with TAG (SEQ ID NO:7) terminal triplet some rising of mRNA levels for cyclins A2 and B1 was characteristic in comparison with other samples, including control sample, which grouped round close values (Fig.1 A, B). In 8 hours was observed the first expression maximum of mRNA for cyclins B1 and E1 (Fig.1 B, C). For cyclin A2 the first maximum has had at 4 hours (Fig. 1 A). The following 12 hours was characterised by depression of gene expression for TAG-terminated variant, as well as for the majority of other samples, including control sample, for which depression was smoother. After 16 hours the minimum falling of expression for cyclins A2, B1, E1 and interleukin-2 was observed. However, mRNA levels of these genes under the influence of TAG-terminated ending have not reached zero value, as for TTA, GTT and GGT variants of the endings of telomeric oligonucleotides. After expression minimum at 16 hours, the growth of mRNA levels with the secondary maximum for cyclins A2 and B1 and insignificant rising for interleukin-2 was observed at 20 hours. Especially brightly the secondary maximum of expression under the influence of TAG-terminated oligonucleotide was displayed for cyclin A2 against its absence for other variants of endings (Figs. 1A). For cyclin E1 the secondary maximum has come after 24 hours of influence (Fig. 1 C).

The variant of telomeric sequence with TTA (SEQ ID NO: 12) terminal triplet reminded action of TAG ending with some specific differences. It confirms the action closeness of the telomeric overhangs, which endings differ in sequence shift by one nucleotide. The fact that TTA ending has not shown essential increase of cyclin expression, which responsible for advance of specific cell cycle phases, such as cyclins A2, E1 and B1, testifies to its primary influence in other phases. First of all it concerns the trigger moment of activation of cell division, which are carried out at transition of G₀ phase to G₁ phase. It has been shown, that in this moment long increases amount of TTA ending of telomeric overhangs in cells. In this connection the action of this ending in other phases of the cell cycle, which has been not relate to the moment of division activation, is defined by its influence on the time of their coming due to regulation of cyclin expression.

The special importance has expression maximum of interleukin-2 at 8 hours under the influence of TTA ending, which more than in 10 times exceeded value of control sample (Fig. 1E). It confirms the perspectivity of use TTA ending of synthetic analogues of telomeric overhangs for cell division stimulation.

The GGT (SEQ ID NO:10) variant of the endings similar to other variants also was in an antiphase to AGG ending, but was characterized by the most later coming of maximums and minimums of expression of studied genes. For dynamics of cyclin A2 and B1 the coming of first expression maximum, as well as secondary, has been shifted. It came at 12 hours for cyclin A2 and at 10 hours for cyclin B1 (Fig.1 A, B). The first minimum of gene expression came at 16 hours as for GGT and TTA terminal triplets. The secondary maximum of expression for all cyclins was lengthened in time and came after 24 hours of lymphocytes treatment by GGT ending. The facts about influence of GGT ending of the telomeric overhangs has been specified by additional experiment, in which concentration of oligonucleotide (GGTTAG)₂GGT was 10 mkM an example 3.

Average position between TTA and GGT variants of the telomeric endings occupied terminal triplet GTT(SEQ ID NO:11). It confirms their closeness and difference due to shifts by one nucleotide to the right or to the left on a length of telomeric sequence.

The GGG (SEQ ID NO: 9) variant of the telomeric endings was intermediate between antiphase AGG variant and other endings. Under its influence the increasing of mRNA level at 16 hours for cyclin B1 and E1 was observed and has coincided with maximum for AGG terminal variant (Figs. 1 B, C). Thus, at the first 12 hours the influence of GGG ending was like influence of terminal triplets TAG, TTA, GTT and GGT, and then became similar to influence of AGG ending. This observation confirms the existence of nucleotide shifts cycle in the telomeric endings, in which the transitions of terminal triplets GGG→AGG, as a result of exonuclease eliminating of 3 '-end nucleotides, is carried out.

By the general observation of influence of described telomeric sequences and their endings on expression of cyclins and interleukin-2, it is visible, that all of them invoke the alterations in mRNA dynamics of these genes. The control sample of cell culture, in which the water was added instead of oligonucleotides, was intermediate between used telomeric sequences. This fact specifies that in native culture of lymphocytes there are all variants of the nucleotide endings of telomeric overhangs at the certain balanced quantitative interrelation. The addition of synthetic analogues of telomeric overhangs to this native interrelation has invoked alterations in expression of cyclins and interleukin-2, and, thus, influenced on the progression of phases of cell division.

### EXAMPLE 2. Suppressing influence of AGG (SEQ ID NO: 8) ending of telomeric nucleotide sequence on the proliferative status of human lymphocytes.

In this experiment the quantity of oligonucleotide (AGGGTT) ₂AGG in final concentration 10 mkM was used. This concentration was twice smaller, than at the first example for estimating possible dose-dependent effect of influence. The moment of the time, corresponding to medium percentage content of AGG ending 12,5%, was chose for oligonucleotide introduction. Close percentages allow comparing the role of AGG ending at close degree for both examples 1 and 2.
Results are presented by curves (Fig. 2), displaying mRNA levels for cyclins A2, B1, D2 and E1 and interleukin-2 after 15 hours of lymphocytes treatment by telomeric oligonucleotides. The shaped line designated minimum of gene expression and the dashed-dot line - expression maximums.

It has been revealed that treatment of lymphocyte culture by synthetic analogues of telomeric overhangs has dose-dependent effect. However, the general character of changes in expression of the studied genes, which relates to expression minimum at 10-12 hours, was conserved and is not dependent on concentration of introduced oligonucleotides. It is showed by a shaped line on curves on Figs. 2. The exception reveals the cyclin D2, which was opposite characterized by excess of mRNA level over a control sample at 10 hours (Fig. 2 C).

As 10 hour's period most brightly showed effect of influence of AGG-terminated oligonucleotide, the repeated analysis of cyclin expression with the same sample cDNA has been made. The results are shown by the bar diagram on Fig. 3, which demonstrates that for the majority cyclins (A2, B1, D3 and E1) was confirmed 10 hour's decrease of mRNA in comparison with a control sample. The expression increase of cyclin D2 against depression of mRNA level for cyclin D3 was observed that probably testifies about delay of the part of cells in Go phase.

Clearly, that 10 mkM concentration depressed the cyclin expression less effectively, than 20 mkM concentration, used in example 1. The curve of cyclines mRNA levels (Fig. 2) conserved the convex form at the first 5 hours retrying dynamics of control sample. Even against the reduced concentration of telomeric oligonucleotide the expressed minimum of mRNA levels for cyclins A2 B1 and E1 at 10-12 hours was observed that testified about supressing function of AGG-terminated DNA ending. The final concentration 20 mkM for AGG-terminated oligonucleotide has the big suppressing action, than 10 mkM concentration. However, the further increase of concentration over 30 mkM not expediently for the therapeutic purposes. Researches shows, that use of 40 mkM concentration of TAG-terminated oligonucleotides already toxically for cells and invokes a culture growth inhibition, in some cases leads to apoptosis and autophagy.

It is important to consider the density of cells in culture or in organism. In this connection it is recommended to count the index of T-oligos quantity per cell. In experiment with 10 mkM concentration of T-oligos were used total eight thousand pmoles of oligonucleotides for six millions cells, that approximately corresponded to 1,33 fmoles per cell. Considering not strongly expressed suppressing action, which was observed at this concentration, it is possible to consider, that such quantity is minimum for therapeutic effect. It is better to use 20 mkM final concentration of oligonucleotide with quantity not less, than 2,7-3 fmoles per cell. However, it is necessary to remember, that this quantity is theoretical and assume the free membrane diffusion of all quantity of oligonucleotides into cells. Actually, infiltration of oligonucleotides into cells depends on rate of membranous diffusion, therefore, not all their quantity is captured by cells. Besides, the influence of introduced synthetic telomeric analogues in cells always overlaps with characteristics of native telomeric overhangs during cycle of terminal nucleotide transitions and depends on phases of cell cycle, in which they are. Thus, for introduction of AGG-terminated oligonucleotides is necessary to choose the moments of the time, corresponding to its minimum percentage content at native Terminal Nucleotide Profile in cells. It defines the efficiency of cell division suppression and cell growth inhibition in anticancer therapy.

In the conclusion of the review about influence of AGG-terminated oligonucleotides it would be important to notice, that its prominent feature is wavy character of changes of cyclines expression, accompanied by suppression at the first 10-12 hours. From the analysis during two experiments, it is possible to draw a conclusion, that the minimum level of mRNA for cyclin A2 existed at 10-12 hours, for cyclin B1 it was a little earlier at 8-10 hours, for cyclin D3 was around 10 hours and for IL-2 - at 12 hours. For cyclin E1, not in all cases, the minimum at 10 hours was observed. For cyclin D2 the mRNA level has slightly decreased at 8 hours.

After period of first minimum of gene expression the subsequent growth of mRNA levels with maximum practically in one period at 16 hours for the majority cyclins (A2, B1, E1) and interleukin-2 was observed. Already at 15 hours for cyclins A2 and E1 and also for IL-2 the excess of mRNA level over control sample was revealed (Fig. 2 A, D, E). It testified, that these cyclins before all others were released from suppressing influence of AGG-terminated T-oligos and allows cells to pass from G₁ phase to synthetic phase of cell cycle. Presumably it is relates to general nuclease influences, acting on oligonucleotides in cells, which leads to its degradations and loss of suppressing properties. Therefore at 15-16 hours after the beginning of influence of AGG-terminated oligonucleotides the expression grows almost equally for all investigated genes (Fig. 1). Only cyclin D2 was characterized by insignificant maximum of expression at 16 hours under the influence of AGG ending.

The shift of first maximum for cyclin B1 expression from 8 hours to 16 hours (Fig. 1 B) shows the prolonging of the coming of mitotic phase of cell cycle and increase of its total duration under the influence of ending AGG. It has led to prolong of the total duration of cell cycle on 8 hours. In comparison with sequence (TTAGGG)₂TTA, at which the cell cycle duration has composed 16 hours, the prolongation of cell cycle under the influence of sequence (AGGGTT)₂AGG has composed 50 % from its total duration. It shows the big prospects of the declared method for controlling of the cell cycle duration.

The AGG-terminated ending of telomeric overhangs, prevailing by the quantity and quantum emission among others in the basic quantum state of nondividing cells, plays important role restraining cyclin expression and cell advance to division. The restraining role of this variant of DNA terminal triplet, which has opposing action in relation to other variants responsible for progression of cell cycle phases can be used effectively in regulation of cell proliferation in the therapeutic purposes. It is shown that use of AGG-terminated variants of T-oligos for division suppression on some orders is more effective than use popular TAG ending. The special meaning possible can have the phosphorothioate-linked (PS) forms of AGG ending protecting it from degradation and prolonging its suppressing action on cell division.

### EXAMPLE 3. Influence of GGT (SEQ ID NO: 10) ending of telomeric nucleotide sequence on the proliferative status of human lymphocytes.

In this experiment the concentration of oligonucleotide (GGTTAG) ₂GGT was 10 mkM. Reduction of T-oligos concentration, in comparison with the first example, was slightly reflected in its influence in spite of the fact, that the culture density remained same and each cell has received the smaller quantity of oligonucleotide. The moment of the time, corresponding to medium percentage content of GGT ending 8,4 %, was chose for oligonucletide introduction. Close percentages allow comparing the role of ending GGT at close degree for both examples 1 and 3.

The results are presented by curves on Fig. 4, where the shaped line designates minimum of gene expression and dashed-dot line - maximums of gene expression. In the period between 2-6 hours of influence the insignificant falling of expression for cyclin B1 and more expressed depression for cyclin A2 was observed (Figs. 4 A, B). Most likely it is relates to native level of cyclin mRNA fluctuation as it occurred synchronously to control sample. Therefore, comparison of this data with the results, received in example 1 (Fig. 1 A, B), will be more correct since 6 hours after beginning of T-oligos influence.

The form of the mRNA curve on the Fig. 4 A testified that for cyclin A2 the shift in time of expression maximum from 10 hours to 12 hours in comparison with control sample was observed. The level maximum of cyclin A2 mRNA at 12 hours is noted by convex shaped line on the drawing. At 18 hours the level minimum of mRNA expression for cyclin A2 was observed, which was similar to minimum at 16 hours on Fig. 1A.

For cyclin B1 the expression maximum at 10 hours was observed, that corresponded to investigation of 20 mkM influence of GGT ending in example 1 (Fig. 4 B, 1 B). However, the expression minimum for cyclin B1 has come at 22 hours, much later, than in example 1, and has coincided with a control sample.

For cylin D2 the additional minimum of mRNA expression at 10 hours and less expressed increase at 18 hours was revealed (Fig. 4 C). Apparently it was continuation of the 16 hour's minimum observed at 20 mkM influence of GGT-terminated oligonucleotide in example 1 (Fig. 1 D). The secondary maximum of cyclin D2 expression was observed at 22 hours as well as for cylin E1 (Fig. 4 C and D).

The mRNA level excess for cyclin E1 over control sample was not common in comparison with example 1 and testified to greater progression of early S phase (Fig. 4 D, 1 C). It witnessed to more successful moment of the T-oligos influence on the cells, which dependent on native Terminal Nucleotide Profile in cells, existing in the moment of T-oligos introduction, which influencing on the progression of terminal nucleotide transitions cycle and the progression of cell cycle. However, despite differences the mRNA curve was conserved and corresponded to curve at 20 mkM influence of GGT-terminated oligonucleotide in example 1 (Fig. 1 C). The characteristic expression minimum between 14 and 18 hours which has coincided with 16 hour's absence of detected mRNA signal in example 1 was observed. Also, the expression maximum for cyclin E1 at 22 hours, which corresponded to increasing of expression after 20 hours of influence in example 1, was revealed.

For interleukin-2, as well as for cyclin A2, some decrease of expression at the first 6 hours took place (Fig. 4 E). But then mRNA level growth was observed up to maximum level at 10 hours, which was twice rising above the control sample. Thus, the new maximum of interleukin-2 expression under the influence of GGT-terminated T-oligos has been found. It is similar to the 8 hour's expression maximum for interleukin-2 observed for TTA and GGG variants of the ending of synthetic telomeric sequences (Fig. 1 E).

In conclusion of the review of influence of studied telomeric oligonucleotide sequences we assumed that each of them specifically influences on the dynamics of expression of the main cyclins defining the coming of maximums and minimums of their mRNA levels. In generalization it is necessary to notice, that for TAG (SEQ ID NO:7) ending the coming of maximums and minimums (first and second) was earlier in comparison to all other endings, including the control sample. It relates to representations, that the terminal triplet TAG is first in a chain of circular transitions of terminal triplet AGG to other triplets under the influence of possible exonuclease eliminating of terminal nucleotides at the 3'-ends of telomeric DNA. In dynamics of cyclin A2 under the influence of TAG-terminated oligonucleotide, the first expression maximum came before others endings already at 4 hours, and for TTA, GGG, GTT endings and control sample it approached at 8 hours.

For GGT-terminated oligonucleotide the coming of first maximum of expression for majority cyclines is shifed in time and came only at 12 hours. For TAG ending the first minimum came between 12 and 16 hours and for TTA, GTT and GGT endings - more later at 16 hours. The mRNA levels of studied genes reduced to zero against smoother depression for control sample. Thus, telomeric sequences with terminal triplets TAG and GGT, as much as possible, differ from each other and are an antagonists, concerning telomeric repeat variation by the shift of whole nucleotide triplet (TAG GGT and GGT TAG).

The special attention is deserved the influence of studied synthetic analogues of telomeric overhangs on gene expression of interleukin-2. The expression maximum of this gene for GGT and possible for GTT terminal triplets is shifted and came at 10 hours while for GGG and TTA endings it came earlier at 8 hours.

As described above, the AGG ending sharply differs from all other endings and has antiphase dynamics of action in relation to them. The minimum of expression for all studied genes under the influence of AGG ending corresponded to maximums for other telomeric endings, and, on the opposite, the maximum of expression under the influence of AGG ending corresponded to minimums for other telomeric endings. The 12 hour's minimum of expression for cyclin A2 under the influence of AGG terminal triplet has coincided with maximum for GGT terminal triplet and the 8 hour's minimum for cyclin B1 has coincided with maximums for TAG, GGG, GTT terminal triplets and for control sample. And on the contrary the 16 hour's minimum of expression for cyclins A2 and B1 under the influence of TAG, GGT, GTT and TTA endings and control sample has coincided with a maximum for AGG triplet ending. In this period the influence of terminal triplet GGG and control sample reminded dynamics of AGG ending action. For cyclin E1 the gradual increase of mRNA level under the influence of AGG terminal triplet was observed against falling of its expression at 16 hours for all other variants of the endings. And only GGG terminal triplet again reminded dynamics of AGG in this period.

In example 2 under the influence of ending AGG for the majority cyclins (A2, B1, D3 and E1) has proved the 10 hour's decrease in comparison with a control sample (Fig. 2, 3). Expression increase for cyclin D2 against depression for cyclin D3 was observed that probably testifies to delay of part of cells in G₀ phase under the influence of AGG ending of telomeric overhang.

In comparison with action of other endings the ending AGG rendered the similar antiphase effect on interleukin-2 expression. Despite visible depressing effect, which was observed at the first 12 hours in example 1 (Fig.1 E) the expression maximum for interleukin-2 in example 2 was found at 5 hours (Fig. 2 E). It proves again that each terminal nucleotide as a part of telomeric overhang defines the coming of specific maximum of expression of the genes controlling cell division. Thus, terminal shifts on one nucleotide leads to alteration in the coming of maximum of gene expression.

For interleukin-2 under the influence of ending AGG the expression maximum at 5 hours was characteristic. Considering communication between shift on one nucleotide in the ending of telomeric oligonucleotide and alteration in the coming of maximum of IL-2 gene expression for ending TAG probably it existed an hour later at 6 hours. The view of the mRNA curves on Fig. 1 E testifies to it where the curve corresponding to terminal triplet TAG not such sloping in comparison with the curve corresponding to triplet AGG. It testifies that the expression maximum for interleukin-2 under the influence of TAG ending comes later than for AGG ending.

The following maximum of IL-2 expression was observed at 8 hours for TTA and GGG endings of telomeric sequence and at 10 hours - for GGT ending. Considering shifts of the endings on one nucleotide the mediate position between terminal triplets TTA and GGT occupies terminal triplet GTT. For it the presumable maximum of IL-2 expression was an interval between 8 and 10 hours at 9 hours.

Thus, it has been positioned that for the AGG ending the earliest coming of first maximum of IL-2 expression at 5 hours was characteristic. Then a series of one nucleotide shifts in endings of telomeric DNA due to exonuclease eliminating of terminal nucleotides invokes elongation of the coming of maximums of gene expression up to 10 hours that corresponds to terminal triplet GGT. It was characterized by the most late coming of the first maximum of IL-2 expression. All process of alteration in the coming of expression maximum for gene interleukin-2, depending on variant of the triplet nucleotide ending of telomeric overhang, is reflected schematically:

AGG (5 h.) → TAG (6 h.) → TTA (8 h.) → GTT (9 h.) → GGT (10 h.) → GGG (8 h.)

Exception of the described schema is the ending GGG, which in spite of the fact, that it differs on the whole triplet from ending TTA, has an identical maximum of IL-2 expression. However, considering nucleotide shifts the ending GGG should have more prolonged maximum than ending GGT. Exclusiveness of telomeric ending GGG most likely is related to its unique fluorescent characteristics. It has been positioned, that GGG and AGG endings have the maximum of FAM-bound fluorescence in green spectrum. It has great role, as these variants of the nucleotide endings of telomeric overhangs are the maximum donors of light energy in green spectrum. The increased level of green fluorescence is characteristic for equilibrium quantum state of nondividing cells. Because the AGG and GGG endings of telomeric overhangs are similar in spectrum of fluorescence, they have maximums of gene expression, which more close in time, than according to schema of terminal nucleotide shifts.

The similar expression maximums have been positioned for cyclin B1 gene. The connection of expression for interleukin-2 and cyclin B1 genes is great importance for understanding of cell cycle progression and cell malignancy. It is positioned that in carcinoma cells the mRNA level of IL-2 at 5-10 times greater in M phase, when the expression of cyclin B1 is high [Reichert T.E is high., Nagashima S., Kashii Y., Stanson J., Gao G., Dou Q.P., Whiteside T.L. Interleukin-2 expression in human carcinoma cell lines and its role in cell cycle progression. Oncogene, 2000, Vol.19, pp. 514-525]. For normal lymphocytes and ceratinocytes this connection has not been shown. However, considering influence of 20 mkM concentration of synthetic overhangs in first example, we revealed the synchronism of expression dynamics for these genes for natural stimulated lymphocytes (Fig. 5). The maximums for AGG ending at 5 hours, for TAG ending for 6 hours and for GGT ending for 10 hours, which received for other time intervals is not displayed in a drawings.

As for terminal triplets TAG, AGG, GGT and GTT (SEQ ID NO:7,8,10,11) the expression maximums for cyclin B1 and IL-2 had on other time intervals than displayed in Fig. 5 their synchronism is shown only by an exterior view of curves. For these endings the synchronous rising of mRNA level for interleukin-2 comparable to growth for cyclin B1 was not displayed on curves. Only for GGG and TTA endings, which maximums had on a construction point of curves at 8 hours, the synchronized maximums for these genes were observed (Fig. 5 C, F). Probably, for other variants of the telomeric endings the expression of cyclin B1 and IL-2 genes are connected in other time intervals.

Thus, in example 1 the expression leveles of mRNA for cyclin B1 and IL-2 have been much connected with each other after 8 hours of TTA and GGG ending's influence. It shows that expression connection of these genes is characteristic for many malignant cells and relates to long prevalence in them of the TTA-terminated telomeric DNA. It has been shown in experiments on studying of Terminal Nucleotide Profiles of telomeric G-strand in leukemic cultures of human lymphocytes. Revealing of this mechanism is important for understanding of process of cancer cell autostimulation to unlimited division by means of activation of interleukin's pathway.

Basing on schema of connected maximums of cyclin B1 and IL-2 expression, the mechanism of opposing action of AGG ending of telomeric overhang, in comparison with other endings, is found out. It most likely is bound by that the maximum for AGG ending comes earlier and has finish before others (about 5 hours) and gets to an antiphase with action of other terminal triplets. Thus, the antiphase dynamics of terminal triplet AGG in comparison with action of other terminal triplets is well shown by mRNA curves (Fig. 1). The revealed feature of ending AGG to enter a counterweight in relation to other endings including TTA is characteristic for the cells activated to division and can use for proliferation suppression.

It is important to notice that cyclins are responsible for transit of late synthetic phase because It activate cyclin-dependent kinase Cdk2 and provide formation of a complex with Cdk1 (a mitosis-stimulating factor) providing cell entrance in mitosis. In this connection there is clear an indisputable influence of the endings of telomeric overhangs not only on preparation of cell division in phases G1, S and G2 through expression regulation of cyclins D, A and E, but also on definition of time of the coming of mitosis by means of control the cyclin B expression. These facts testify that variants of the terminal triplets of telomeric overhangs are in the cells in specific quantitative interrelation, which variates during a cell cycle. Thus, it controls the transit of phases of a cell cycle, in particular, the coming of mitosis through regulation of gene expression relates to cell division.

The control sample containing only native overhangs in a physiological interrelation was intermediate on dynamics of changes of gene expression of cyclins and interleukin-2 (Fig.1). Introduction into cells of artificial analogues of telomeric overhangs, which have various sequences and various endings, has invoked alterations in dynamics of the genes, regulating cell proliferation. In this case the expression maximums of the studied genes under the influence of various sequences were not overlapped. For each hour there was one maximum of mRNA level for cyclins and interleukin-2 genes under the influence of one variant of telomeric sequences. The uniqueness of each variant of the telomeric overhang endings, which defines the unique maximum of gene expression, responsible for progression of cell cycle phases and cell division regulating consists in it. The exception consists in identical maximums of interleukin-2 expression under the influence of TTA and GGG endings of synthetic telomeric overhangs.

Thus, definite selection of telomeric nucleotide sequences and time of its introduction in cells allows to specific influence on the gene expression, which regulate cell division and target control of the cell proliferative status. It defines new prospects in understanding of mechanisms of the beginning and controlling of cell division and also the cancer malignization of cells due to reduction of cell cycle duration. It has been proved that cancer cells are characterized by the broken regulation mechanism of quantitative interrelation of variants of the nucleotide endings of telomeric G-overhangs that leads to reduction of the time of mitosis coming. This molecular mechanism of cell regulation was named "Nucleotide Clock" and relates to cycle of terminal nucleotide transitions, which leads to cyclic alteration of nucleotide endings of human telomeric DNA. In this connection the cancer cells have broken Nucleotide Clock and altered ratio of G-strand terminal nucleotides during the time.

Hereby it is expedient to introduce the Terminal Nucleotide Profiling of telomeric DNA and the studies of fluorescent and spectral characteristics of telomeric overhangs into diagnostic practice of medical institutions. Carrying out of similar diagnostics will allow obtaining the greatest therapeutic effect from synthetic analogues of telomeric sequences by means of the declared method. Considering that the terminal triplet's ratio in telomeric DNA is important for definition of cell proliferative status, the Correcting Profiles of its nucleotide endings are composed for making of trigger conditions for cell division activation or suppression. Example 4 can be like the example of composing such Correcting Profiles in anticancer therapy.

### EXAMPLE 4. Composing the Correcting Profile of the triplet nucleotide endings of human telomeric DNA for the purpose of cell division suppression in leukemic cultures Jurkat and K-562.

Analysis of the consequent nucleotide profiles in form of Terminal Nucleotide Curves allowed revealing the time points of effective introduction of telomeric oligonucleotides into cells. Initial Terminal Nucleotide Profile and corresponding to him Correcting Profile must be synchronous and correspond to one moment of the time in cycle of terminal nucleotide transitions of telomeric DNA. In this connection the Terminal Nucleotide Profiles for G-strand of telomeric DNA after time synchronization of cells in mitosis phase under the influence of colchicine have been received for each leukemic cultures (Fig .6 A - Jurkat, Fig.6 B - K-562). They testified that the significant disorders of the processes controlling terminal nucleotide transitions on the 3'-ends of telomeric overhangs were characteristic for the both cell cultures.

For the normal lymphocytes characteristically the periodic presence of the equilibrium balanced profile of terminal nucleotides of telomeric overhangs in main phases of cell cycle excepting initial G1 phase marking trigger process of cell division activation. In this connection the specific ratio of synthetic analogues of the telomeric DNA endings have been calculated for each leukemic culture, which necessary for restore equilibrium balanced profile of terminal nucleotides in an average quantitative interrelation: TAG (18 %), AGG (26 %), GGG (7 %), GGT (5 %), GTT (26 %), TTA (18 %). The received Correcting Profiles are presented by diagrams of percentage content of terminal triplets on Fig. 7 A for Jurkat culture and on Fig.7 B for culture K-562.

Optimum concentration of telomeric oligonucleotides according to the positioned percentage content should be selected empirically and be defined by quantitative presence of TTA ending of telomeric overhangs in cells-recipients. It is necessary to compose of Correcting Profiles according the rule: the initial excessive amount of terminal triplet TTA in resulting profile is taken like a norm, compounding 18 %, in relation to which, add concentrations of other triplet endings of telomeric overhangs. For culture Jurkat amount of TTA triplet in initial profile has compounded 82,3 %. At composition of the resulting profile, which formed at addition of oligonucleotide concentration in a percentage ratio presented on Fig. 7 A the content of TTA ending has compounded 18 %. Thus, the total concentration quantity of the synthetic analogues of telomeric overhangs, added to cells, more than initial quantity of the native overhangs in 3,6 times. For culture K-562 the quantity of TTA ending, which compounded 57,5 % in initial profile, also was taken like 18 % content in resulting profile. According it, the added quantities of the triplet endings of telomeric oligonucleotides were in 2,2 times more, than initial quantity of the native telomeric overhangs in cells and correspond to the percentage, presented on Fig. 7 B.

The augmentation of telomeric overhangs in cells in the specified limits does not lead to the cytotoxic effects relating to their overabundance. However, at the calculation of introduced oligonucleotides concentration, it is necessary to accord with no more than 10-20 mkM limit of final total concentration of all variants of the telomeric endings. The augmentation of concentration, more than 40 micromoles, can invoke the cytotoxic effects, related to their overabundance. Influence of telomeric oligonucleotide mixtures, according declared method, is not relate to multiple excess of their physiological concentration and is not a signal of DNA damage, leading to arrest of cell cycle. The telomeric oligonucleotide mixtures are designed for correction of Terminal Nucleotide Profiles of telomeric DNA at specific moment of time in treated cells and their return to controllable conditions of division.

Due to impossibility of carrying out of the individual Terminal Nucleotide Profiling of telomeric DNA for malignant cells, it is recommended to use the influence by AGG (SEQ ID NO:8) terminated oligonucleotides as the most important for nucleotide profile of nondividing cells. Use of sequences on the basis of full-length repeat GTTAGG (SEQ ID NO: 2) is perspective as they have the maximum fluorescence in green spectrum. It is very important for conditions of the quantum state of nondividing cells, for which characteristically the simultaneous presence of green and red channels of the telomeric overhang fluorescence. Besides this quantum effect can be enhanced by incorporation of the green fluorescent label and the quencher of red fluorescence. Probably incorporation in oligonucleotides the phosphorothioate-linked groups will lead to blocking of eliminating of terminal nucleotides and prolonging its therapeutic action. Application of various modifications of AGG-terminated oligonucleotides allows not only prolonging their therapeutic action, but also raising its specificity.

The distinctive feature of the declared method is possibility of designing of the various telomeric sequences, which differ not only in the endings, but also in the sequences of telomeric repeat. It is important for achievement of the maximum therapeutic effect. The understanding of a role of differences of telomeric repeat sequences in cell division regulation lies at the heart of the declared method. Depending on variations of the nucleotide endings of 3' and 5'-termini of telomeric DNA the set of its G-overhangs represents the heteromorphic population differing in nucleotide composition of telomeric repeats. According to 5'-terminus of the C-strand of telomeric DNA, the sequence of each single-strand overhang of G-strand represents the n-th number of one of six variants of full-length repeats, presented in SEQ ID NO:1-6. Thus, G-overhangs can terminate in not full-length repeat and differ in the triplet endings, presented in SEQ ID NO:7-12.

The use of variants of telomeric repeat sequences and its endings, which have specific action on expression of the genes, controlling cell division lies at the heart of declared invention. In examples of invention realization it has been shown, that terminal nucleotides play a huge role in regulation of the coming of expression maximums for cyclin genes, which control the coming of cell cycle phases. In this connection at the designing of telomeric oligonucleotides it is necessary to consider not only their ending, but also their initial nucleotides. They define the sequence of telomeric repeat, which defines their basic spectral fluorescent properties.

It is shown that various variants of telomeric repeat of human DNA have the unique spectral characteristics, which relate to specific balance of red and green colours of their resulting fluorescence. The basic fluorescence specificity of telomeric oligonucleotide is defined by sequence of the telomeric repeat defined by first six nucleotides since 5 '-end. The research of spectral characteristics of the telomeric sequences in green spectrum of the FAM-bound fluorescence testifies to it (Fig. 8). The diagrams show a fluorescence growth of water solution of free carboxyfluorescein dye after addition of telomeric oligonucleotide samples. Thus, comparison of diagrams on Fig. 8 has shown the value difference of fluorophor-bound fluorescence between the sequences, consisting of two full-length repeats (A) and the sequences, terminating in the third not full-length repeat, in the triplet (B). In this connection the special role of terminal triplets of telomeric overhangs in cell division regulation through control of intensity of the green channel of their resulting fluorescence in cells becomes clear.

In cell genome the telomeric repeat GTTAGG (SEQ ID NO: 2), having a high level of green fluorescence, which characteristic for nondividing state of cells, should provide the basic functions of division suppression. It is reached due to suppressing influence on expression of cyclin genes and prolonging of time of the coming of cell cycle phases. For sequence (AGGGTT)₂AGG its basic spectral properties relate to lowered level of green fluorescence has defined by telomeric repeat AGGGTT (SEQ ID NO:5). Nevertheless this telomeric sequence has conserved the antiphase action in relation to other variants of the telomeric endings and has shown suppressing influence on expression of cyclin genes at the first 10-12 hours of lymphocytes treatment. Thus, the influence of the T-oligos sequences, terminating in not full-length telomeric repeat, has shown a primary role of the triplet endings in cell division regulation. The choice of the initial nucleotides on the 5'-end of telomeric sequences allows regulating the sequence of the basic telomeric repeat among SEQ ID NO: 1-6 and obtaining the maximum effect from the used triplet ending. The efficiency of such sequence variants should be revealed experimentally.

For sequence (TTAGGG)₂TTA the basic telomeric repeat is sequence TTAGGG (SEQ ID NO:3), which defines its spectral characteristics, relating to high green fluorescence. Thus, the ending TTA has invoked some decrease of green fluorescence of this telomeric sequence in comparison with oligonucleotide, consisting of two full-length repeats (Fig. 8). In this connection there is clear a role of the nucleotide endings of the full-length telomeric repeats, which are a part of last not full-length repeat.

The sequence (TTAGGG)₂TTA has not revealed the expressed influence on the cell division stimulation. On the one hand it can be relate to its fluorescence in green spectrum and inadequacy to quantum state of cells activated to division and related to prevalence of red fluorescence. On the other hand it can be defined by influence of this sequence not at the moment of transition G₀-G₁ phases at activation of cell division, when it has the maximum stimulating influence. Nevertheless, the use of this telomeric sequence has proved basic role of TTA ending of telomeric overhangs in cell division stimulation through the coming of interleukin-2 overexpression after 8 hours of T-oligos treatment (Example 1, Fig. 1 E). The result of its stimulating effect was acceleration of progression of the cell cycle phases and its most shortened duration. The close zero level of cyclin D2, against absence of others cyclins after 16 hours of influence, testifies about it (Fig.1 A-D). Thus, it is necessary to notice the synchronizing role of ending TTA, leading to, that all proliferating cells have finished its division at 16 hours after beginning of T-oligos treatment.

It is interesting that the sequence (GGGTTA)₂GGG, presented by two full-length repeats GGGTTA (SEQ ID NO:6), has revealed the same 8 hour's maximum of IL-2 expression. Probably in this case the sequence of the basic telomeric repeat GGGTTA has played the leading part in responsibility for activation of cell division. Also the ending GGG is related to the cell state activated to division. The circular periods of augmentation of GGG ending's content testify to it in cells after the trigger moment of division activation relating to long maximum prevalence of TTA ending of telomeric overhangs.

In this connection it has been revealed that the oligonucleotides consisting of full-length telomeric repeats will be more effective in comparison with described telomeric sequences. They conserve the possible expressed differences of spectral properties of the nucleotide endings corresponding to the purposes of influence on the cell proliferative status. In particular for the cell division suppression it is better to use the sequences consisting of n-th number of full-length repeat GTTAGG (SEQ ID NO: 2).

For cell division activation it is expedient to use the sequences consisting of n-th number of full-length repeat GGGTTA (SEQ ID NO: 6). These sequences on the one hand will provide the stimulating influence of ending TTA on cell division. On the other hand due to prevalence of red fluorescence it will completely correspond to the quantum state of cells activated to division. In this connection the investigated sequence (TTAGGG)₂TTA is less effective in comparison with sequences, consists of full-length repeat GGGTTA.

These criteria essentially distinguish a declared method from the last conception of application of synthetic analogues of telomeric DNA, which based on understanding of their role only as signals of DNA damage leading to arrest of cell division. The great importance for the declared invention is the understanding of mutual relations between separate telomeric overhangs in cells as elements of the total quantum resonant system. Thus, the influence on cells by synthetic analogues of telomeric overhangs is perceived as modification of complex system of molecular and quantum communications. Thus, the special importance is the moment of introduction of correcting oligonucleotide mixtures into cells, which is related to formation of Terminal Nucleotide Profiles of telomeric overhangs specific to the concrete moment of the time and current phase of cell cycle.

The antiphase dynamics of AGG ending of telomeric overhangs plays the special role in comparison with other variants of the endings. This variant of the ending at use of the full-length sequences of telomeric repeat GTTAGG (SEQ ID NO: 2) composed the basis of influence on the cell proliferative status for purpose of division suppression. In anticancer therapy the similar analogues of telomeric sequences will be more effective in comparison with used earlier 11-nt form GTTAGGGTTAG. Thus, their efficacy of action will depend on what moment of the time and what phases of cell cycle it is carried out.

The introduction of sequences on the basis of telomeric repeat GTTAGG (SEQ ID NO:2) with specific terminal triplet AGG (SEQ ID NO:8) will have the suppressing action at the moment of division activation. It is relate to antiphase action of this variant of the ending along with TAG (SEQ ID NO:7) and GGG (SEQ ID NO:9) endings on nonequilibrium unbalanced profile of the terminal nucleotides of telomeric overhangs differed by long prevalence of TTA ending. In healthy cells this process leads to inhibition of trigger transition G₀ phase to G₁ phase of cell cycle and to accumulation of cells in resting phase.

In malignant cells the influence of AGG, TAG and GGG endings in specific interrelation at concrete moment of the time invokes not only blocking G₀→G₁ transition at division activation. But also it promotes the formation of equilibrium balanced profile of the terminal nucleotides of telomeric overhangs in certain moment of the time and leads to further realization of normal progression and duration of cell cycle. Besides this influence it provides the switching-off the embryo-specific program of development for cancer cells and carries out their return to tissue-specific specializations.

After the trigger moment of division activation, when the Terminal Nucleotide Profile of telomeric DNA in cells is became equilibrium balanced, the addition of various telomeric sequences leads to alterations in the coming of phases of preparation for division and cell division. The influencing specificity of the various endings and nucleotide variations of telomeric repeat on the coming of expression maximums of the genes controlling cell division has been shown in the first three examples of the invention. For the first time the influencing possibility of specific sequences of telomeric DNA on the time of the coming of cell division and cell cycle phases and on the duration of cell cycle has been shown through regulation of cyclin gene expression.

It is important to notice that during the progression of the cell division related to positioned equilibrium profile of telomeric terminal nucleotides the influence of AGG ending as well as other variants of terminal triplets is related to influence on progression of mitosis phases. At this stage the function of AGG-terminated oligonucleotide differ from function at the division activation, as this ending is capable to prevent, but not to stop already begun cycle of division.

If preparation for division has already begun the AGG ending as well as other endings regulates its progression. It has been shown that it defines the most prolonged coming of synthetic phase of preparation for division in 16-18 hours from the beginning of cell cycle. Dynamics of gene expression for cyclins A2 and E1 testified to it in example 1 (Fig.1 A and C). Full conformity of this fact with normal duration of lymphocyte cell cycle defines a huge role of the AGG-terminated telomeric overhangs in regulation of this process. In this connection there is clear the strategy of cancer cells related to decrease or full reduction of ending AGG for achievement of shortening of cell cycle duration. The deficiency compensation of the AGG-terminated telomeric overhangs composed a basis of new approach in DNA-therapy of oncologic diseases. The cell proliferation suppression is related to antiphase dynamics of AGG ending's influence on expression of cyclins and interleukin-2 genes in relation to action of other telomeric endings. It is related to that all other endings represent certain stages of transformation of initial ending AGG in a cycle of single nucleotide transitions of terminal triplets: AGG→TAG→TTA→GTT→GGT→GGG→AGG. Thus, each nucleotide shift and each ending are characterized by the specific time of the coming of expression maximums of the genes, controlling cell division.

### INDUSTRIAL APPLICABILITY

The invention can find the application in public health services and scientific institutions. The description of invention realization shows the possibility of application of the declared method in medical and research establishments for target controlling of the proliferative status of human cells.

In the declared method open forms of telomeric overhangs are accepted not only, as a DNA damage signal about disruption of the loopback organization of telomeric DNA, which start reparative processes and blocking a cell cycle. First of all it accepted as the special signals of molecular and quantum-optical nature, which are in certain dynamical interrelation, controlling cell life activity. The telomeric DNA is dynamical system, in which is carried out the continuous synchronized process of changes of its triplet nucleotide endings, composing the terminal nucleotide transitions cycle. Thus, the use of carefully selected concentration of the telomeric oligonucleotides, which are according to physiological level of telomeric overhangs in cells, is important. The application of physiologically adapted concentration of the synthetic telomeric oligonucleotides, less than 40 mkM final concentrations in medium, allows avoiding their toxic-like excess, which can lead to arrest of cell cycle. Thus, the synthetic sequences enter in interaction with native overhangs, forming uniform system of quantum-optical regulation of cell genome and processes of cell life activity, including division.

Introduction into cells of excessive amount of synthetic analogues of telomeric overhangs as earlier leads to breakage of the delicate mechanism of genome activity. Certainly in this case the experiments have the result, but different from the operating by concentration, nucleotide variations of telomeric repeat and the endings of telomeric overhangs. The declared method is based on these new principles of influence on the proliferative status of cells by means of specific nucleotide sequences of G-strand of human telomeric DNA. They allow raising the specificity and efficiency of telomeric DNA-therapy by means of the declared sequences and opening wide prospects of its use in various areas of medicine.

It is expedient to introduce the Terminal Nucleotide Profiling and Monitoring of telomeric DNA into diagnostic practice of medical institutions. It allows obtaining the specific Terminal Nucleotide Profiles at current moment of the time and composing the corresponding Correcting Profiles of nucleotide endings and correcting oligonucleotide mixtures for patients. Analysis of the consequent profiles in view of Terminal Nucleotide Curves allows revealing the time points of effective introduction of telomeric oligonucleotides into cells. Initial Terminal Nucleotide Profile and corresponding to him Correcting Profile must be synchronous and correspond to one time point in terminal nucleotide transitions cycle of telomeric DNA. Thus, the efficiency of T-oligos treatment depends on the moment of Correcting Profiles introduction into cells, which corresponds to current native Terminal Nucleotide Profiles in cells-recipients.

Carrying out of preliminary diagnostics of terminal nucleotides will allow obtaining the greatest therapeutic effect from synthetic analogues of telomeric sequences. Considering that the terminal triplet's ratio in telomeric DNA is important for definition of cell proliferative status, the Correcting Profiles of its nucleotide endings can compose for making of trigger conditions for cell division activation or suppression at certain moment of the time.

For individual cells the Correcting Profiles for purpose of AGG ending's compensation at corresponding moment of the time can apply in anticancer therapy. Prolonging of the coming of mitotic phase of cell cycle and increase of its total duration under the influence of AGG-terminated telomeric sequence was shown by the shift of first maximum for cyclin B1 expression from 8 hours to 16 hours (Fig. 1 B). It has led to prolong of the total duration of cell cycle on 8 hours. In comparison with sequence (TTAGGG)2TTA, at which the cell cycle duration has composed 16 hours, the prolongation of cell cycle under the influence of sequence (AGGGTT)2AGG has composed 50 % from its total duration. This fact shows the big prospects of the declared method for controlling of the total duration of cell cycle. The use of Correcting Profiles, consisting of prevalent amount of AGG-terminated telomeric sequences, will allow returning cancer cells to normal duration of cell cycle. The use of prevalent amount of TTA-terminated telomeric sequences will allow to accelerate the coming of the cell cycle phases, specifically, mitotic phase, and shorten the cell cycle duration, which can apply in maintenance of immune and regenerative status of the human.

For the first time the unique combinations of nucleotide variations of telomeric repeat with specific triplet endings of telomeric sequences allows to use them not only for cell growth inhibition, but also for stimulation of cell proliferation and controlling of time of the coming of specific phases of cell cycle, including resting phases, for which the realization of tissue-specific functions of cells and tissues is characteristic. In this connection the specific nucleotide sequences of G-strand of human telomeric DNA can be used as a part of drug preparations in oncology, immunology, gerontology, transplantology or like medical cosmetic products in cosmetology, dermatology, which are designed for target control of cell division and maintenance of tissue-specific functions of cells.

## Claims

1. A method for influence on the cell proliferative status, including introduction in medium, surrounding cells, and the subsequent infiltration into them of the specific nucleotide sequences of single-strand overhangs of G-strand of human telomeric DNA, which are differed by variations of nucleotide sequence of telomeric repeat and the triplet endings, and applied in final concentration, which do not lead to cell-inhibiting excess and DNA-damage signaling of cell cycle arrest, and designed for purposes:
a) suppression or stimulation of cell division, due to regulating of time of the coming of certain phases of cell cycle and his total duration;
b) achievement of the controllable profile of gene expression, relating to cell division and activation or suppression of tissue-specific and embryo-specific programs of cell development.

2. A method as claimed in claim 1, wherein the specific nucleotide sequences of G-strand of human telomeric DNA, presented by the native telomeric overhangs, extracted from cells, which synchronised on certain stage of cell cycle and being donors of the proliferative information for cells-recipients.

3. A method as claimed in claim 1, wherein the synthetic deoxyribo-oligonucleotides of various length, phosphorylated or dephosphorylated on 5 '-end, applied in physiologically adapted concentration, which are not leading to arrest of cell cycle because of their overabundance, are used as nucleotide sequences of G-strand of human telomeric DNA.

4. A method as claimed in claim 3, wherein the synthetic analogues of nucleotide sequences of G-strand of human telomeric DNA consist of n-th number of full-length sequences of the telomeric repeats from among the variants, presented in SEQ ID NO: 1-6.

5. A method as claimed in claim 3, wherein the synthetic analogues of nucleotide sequences of G-strand of human telomeric DNA consist of n-th number of full-length sequences of the telomeric repeats, specified in SEQ ID NO: 1-6, and terminating in not full-length repeat with the terminal triplets, presented in SEQ ID NO: 7-12.

6. A method as claimed in claim 4 or 5, wherein the specified synthetic analogues are applied to controlling of time of the coming of certain phases of cell cycle through regulation of the coming of maximums and minimums of gene expression, related to progression of cell cycle and cell division.

7. A method as claimed in claim 4 or 5, wherein the specified synthetic analogues are applied as a part of the correcting mixture, consisting from presented in SEQ ID NO: 7-12 endings of the G-strand of human telomeric DNA, composed on the preliminary definition of Terminal Nucleotide Profiles of telomeric overhangs in cells and designed for correction these native profiles, corresponding to current moment of the time and certain phases of cell cycle.

8. A method as claimed in claim 3, wherein the nucleotide sequences of G-strand of human telomeric DNA, consisting of n-th number of full-length sequence of telomeric repeat SEQ ID NO: 2 or sequences of other variants of telomeric repeat from SEQ ID NO:1,3,4,5,6, terminating in not full-length repeat with terminal triplet SEQ ID NO:8, are used for the cell division suppression due to prolongation of the coming of mitotic phase of cell cycle and his total duration.

9. A method as claimed in claim 8, wherein the specified sequences are used for cell division suppression, as a part of the correcting mixture, consisting of other triplet endings of G-strand of human telomeric DNA, presented in SEQ ID NO: 7,9,10,11,12, and composed on the preliminary definition of Terminal Nucleotide Profiles of the native telomeric overhangs in cells-recipients.

10. A method as claimed in claim 8 or 9, wherein the specified nucleotide sequences are used for suppression of embryo-specific program and activation of tissue-specific program of cell development, including the achievement of controllable profile of gene expression, relating to their realisation.

11. A method as claimed in claim 3, wherein the nucleotide sequences of G-strand of human telomeric DNA, consisting of n-th number of full-length sequence of telomeric repeat SEQ ID NO: 6 or sequences of other variants of telomeric repeat from SEQ ID NO: 1-5, terminating in not full-length repeat with terminal triplet SEQ ID NO: 12, are used for the cell division stimulation due to shortening of the coming of mitotic phase of cell cycle and his total duration.

12. A method as claimed in claim 11, wherein the specified sequences are used for cell division stimulation, as a part of the correcting mixture, consisting of other triplet endings of G-strand of human telomeric DNA, presented in SEQ ID NO: 7-11, and composed on the preliminary definition of Terminal Nucleotide Profiles of the native telomeric overhangs in cells-recipients.

13. A method as claimed in claim 11 or 12, wherein the specified nucleotide sequences are used for suppression of tissue-specific program and activation of embryo-specific program of cell development, including the achievement of controllable profile of gene expression, relating to their realization.

14. A method as claimed in claim 3, wherein the synthetic analogues of nucleotide sequences of G-strand of human telomeric DNA contain the modifications, presented by terminal or internal incorporation of fluorescent label or fluorescent quencher, designed for intensifying or suppression of fluorescence at certain light spectrum and correction of natural quantum saturation of telomeric overhangs in cells for the quantum-optical regulation of cell division.

15. A method as claimed in claim 14, wherein the fluorophores of dark blue or green fluorescence spectrum or fluorescent quenchers of orange-red or red spectrum are used for cell division suppression and activation of the tissue-specific genetical program of cell development.

16. A method as claimed in claim 14, wherein the fluorophores of orange-red or red fluorescence spectrum or fluorescent quenchers of dark blue or green spectrum are used for cell division stimulation and activation of the embryo-specific genetical program of cell development.

17. A method as claimed in claim 15 or 16, wherein the specified fluorophores and quenchers are presented by synthetic or natural molecules, including native metabolites of human cells.

18. A method as claimed in claim 15 or 16, wherein the specified fluorophores and quenchers are not used for chemical modification of telomeric nucleotide sequences, but are introduced into cells-recipients in the form of free components and carry out the correction of their total quantum saturation.

19. A method as claimed in claim 3, wherein the synthetic analogues of telomeric sequences contain the modifications, presented by phosphorothioate-linked groups or similar linkages, designed for avoidance of their nuclease degradations and end eliminating of terminal nucleotides, relating to transitions in other forms of the endings, and providing prolongation of their therapeutic action.

20. A method as claimed in claim 3, wherein the synthetic analogues of telomeric sequences contain the modifications, presented by photosplitting linkages (spacers), designed for the target hydrolysis of certain internucleotide linkages under the light of specific wave length and the controlled appearance of the specific triplet endings of telomeric overhangs at the concrete moment of the time, according to terminal nucleotide transitions cycle.

21. A method as claimed in claim 1, wherein the specific nucleotide sequences of G-strand of human telomeric DNA are used like a part of: a) drug preparations in oncology, immunology, gerontology, transplantology and other areas of medicine, b) medical cosmetic products in cosmetology and dermatology, which are designed for controlling of cell division and maintenance of tissue-specific functions of cells.

22. A method as claimed in claim 1, wherein the wave equivalents (replicas) are used as specific nucleotide sequences of G-strand of human telomeric DNA in forms of: a) electromagnetic waves, including light and radio-waves spectrums, b) sound waves, c) fields, formed by oscillatory or rotary movements of material particles, torsion and other fields of vacuum and quantum nature, waves of holographic and nonlinear soliton origin, consisting of the certain resonance frequencies, influencing on the cells-recipients and human organism.


SEQUENCE LISTING
<110> CHIRYASOVA, Elena Andreyevna
<120> Method for influence on the cell proliferative status using specific G-strand nucleotide
sequences of human telomeric DNA
<130> CHIROLIG-1-PCT
<150> RU 2012153541/10
<151> 2012-12-12
<160> 12
<210> 1
<211> 6
<212> DNA
<213> Artificial Sequence
<220>
<223> Basic sequence variant of the G-strand repeat of human telomeric DNA for composition
of the specific nucleotide sequences, consisting of its n-th number, and used for influence on
the cell proliferative status
<400> 1
ggttag
<210> 2
<211> 6
<212> DNA
<213> Artificial Sequence
<220>
<223> Basic sequence variant of the G-strand repeat of human telomeric DNA for composition
of the specific nucleotide sequences, consisting of its n-th number, and used for influence on
the cell proliferative status
<400> 2
gttagg
<210> 3
<211> 6
<212> DNA
<213> Artificial Sequence

<220>

<223> Basic sequence variant of the G-strand repeat of human telomeric DNA for composition
of the specific nucleotide sequences, consisting of its n-th number, and used for influence on
the cell proliferative status

<400> 3

ttaggg

<210> 4

<211> 6

<212> DNA
<213> Artificial Sequence

<220>

<223> Basic sequence variant of the G-strand repeat of human telomeric DNA for composition
of the specific nucleotide sequences, consisting of its n-th number, and used for influence on
the cell proliferative status

<400> 4

tagggt

<210> 5

<211> 6

<212> DNA
<213> Artificial Sequence

<220>

<223> Basic sequence variant of the G-strand repeat of human telomeric DNA for composition
of the specific nucleotide sequences, consisting of its n-th number, and used for influence on
the cell proliferative status

<400> 5

agggtt

<210> 6

<211> 6

<212> DNA
<213> Artificial Sequence

<220>

<223> Basic sequence variant of the G-strand repeat of human telomeric DNA for composition
of the specific nucleotide sequences, consisting of its n-th number, and used for influence on
the cell proliferative status

<400> 6

gggtta

<210> 7

<211> 3

<212> DNA
<213> Artificial Sequence

<220>

<223> Triplet nucleotide ending of the specific G-strand sequences of human telomeric DNA,
used for influence on the cell proliferative status

<400> 7

tag

<210> 8

<211> 3

<212> DNA
<213> Artificial Sequence

<220>

<223> Triplet nucleotide ending of the specific G-strand sequences of human telomeric DNA,
used for influence on the cell proliferative status

<400> 8

agg

<210> 9

<211> 3

<212> DNA
<213> Artificial Sequence

<220>

<223> Triplet nucleotide ending of the specific G-strand sequences of human telomeric DNA,
used for influence on the cell proliferative status

<400> 9

ggg

<210> 10

<211> 3

<212> DNA
<213> Artificial Sequence

<220>

<223> Triplet nucleotide ending of the specific G-strand sequences of human telomeric DNA,
used for influence on the cell proliferative status

<400> 10

ggt

<210> 11

<211> 3

<212> DNA
<213> Artificial Sequence

<220>

<223> Triplet nucleotide ending of the specific G-strand sequences of human telomeric DNA,
used for influence on the cell proliferative status

<400> 11

gtt

<210> 12

<211> 3

<212> DNA
<213> Artificial Sequence

<220>

<223> Triplet nucleotide ending of the specific G-strand sequences of human telomeric DNA,
used for influence on the cell proliferative status

<400> 12

tta